# EUROPEAN PATENT APPLICATION

(11) **EP 0 708 097 A1**
(43) Date of publication of application: **24.04.1996**
(21) Application number: 95115675.1
(22) Date of filing: 05.10.1995
(51) Int. Cl.: C07D 257/04, A01N 47/38, A01N 43/713

(54) **Herbicidally active tetrazolinones**

(30) Priority: 18.10.1994 JP 277189/94; 18.10.1994 JP 277191/94; 18.10.1994 JP 277193/94; 19.10.1994 JP 278583/94; 19.10.1994 JP 278580/94
(71) Applicant: NIHON BAYER AGROCHEM K.K., Tokyo 108 (JP)
(72) Inventor: Goto, Toshio, Shimotsuga-gun, Tochigi (JP); Ito, Seishi, Oyama-shi, Tochigi (JP); Watanabe, Yukiyoshi, Hasuda-shi, Saitama (JP); Narabu, Shin-ichi, Yuki-shi, Ibaraki (JP); Yanagi, Akihiko, Tochigi (JP)
(74) Representative: Linkenheil, Dieter

(57) **Abstract**

Novel tetrazolinones represented by the formula 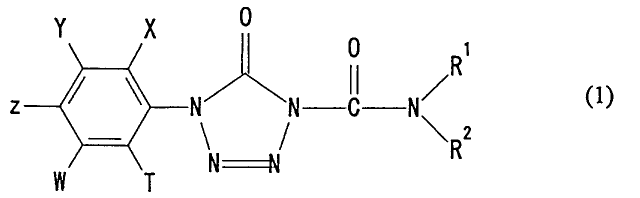
wherein
- X is: hydrogen, halogen, C₁₋₄ alkyl, C₁₋₂ alkoxy, C₁₋₂ alkylthio, C₁₋₂ alkylsulfonyl, C₁₋₂ alkylsulfinyl or halogeno-C₁₋₂ alkoxy,
- Y is: hydrogen, halogen, C₁₋₄ alkyl or trifluoromethyl,
- Z is: hydrogen, halogen, C₁₋₄ alkoxy or halogeno-C₁₋₂ alkoxy,
- W is: hydrogen, halogen, C₁₋₄ alkyl or trifluoromethyl,
- T is: hydrogen, halogen or C₁₋₄ alkyl,
- R¹ is: C₁₋₅ alkyl,
- R² is: C₃₋₇ cycloalkyl which may optionally be substituted by alkyl, C₅₋₆ cycloalkenyl, a bridged alicyclic group or C₄₋₆ branched alkyl,
and
R¹ and R² may form an optionally substituted cyclic group, together with the nitrogen atom to which they are bonded,
with the proviso that where R² is C₄₋₆ branched alkyl, then the total number of the carbon atoms of R¹ and R² is 7 or 8, and
with the exception of the case where
X is chlorine or bromine,
Y, Z and W are hydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8,
a process for their preparation and their use as herbicides.

## Description

The present invention relates to novel tetrazolinones, to a process for their preparation and to their use as herbicides.

It has already been disclosed that a certain group of tetrazolinone derivatives has herbicidal function (see EP-A-0146279; and further:
U.S. Patents Nos. 4,618,365; 4,826,529; 4,830,661; 4,956, 469; 5,003,075; 5,019,152; 5,342,954; 5,344,814; 5,347, 009; 5,347,010 and 5,362,704).

There have now been found novel tetrazolinones of the formula (I) 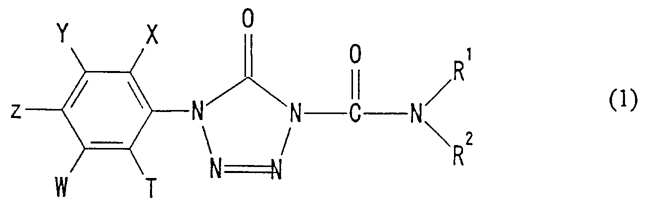
wherein
- X is: hydrogen, halogen, C₁₋₄ alkyl, C₁₋₂ alkoxy, C₁₋₂ alkylthio, C₁₋₂ alkylsulfonyl, C₁₋₂ alkylsulfinyl or halogeno-C₁₋₂ alkoxy,
- Y is: hydrogen, halogen, C₁₋₄ alkyl or trifluoromethyl,
- Z is: hydrogen, halogen, C₁₋₄ alkoxy or halogeno-C₁₋₂ alkoxy,
- W is: hydrogen, halogen, C₁₋₄ alkyl or trifluoromethyl,
- T is: hydrogen, halogen or C₁₋₄ alkyl,
- R¹ is: C₁₋₅ alkyl,
- R² is: C₃₋₇ cycloalkyl which may optionally be substituted by alkyl, C₅₋₆ cycloalkenyl, a bridged alicyclic group or C₄₋₆ branched alkyl,
and
R¹ and R² may form an optionally substituted cyclic group, together with the nitrogen atom to which they are bonded,
with the proviso that where R² is C₄₋₆ branched alkyl, then the total number of the carbon atoms of R¹ and R² is 7 or 8, and
with the exception of the case where
X is chlorine or bromine,
Y, Z and W are hydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8.

The compounds of the formula (I) can be obtained by a process in which
a) compounds of the formula (II) 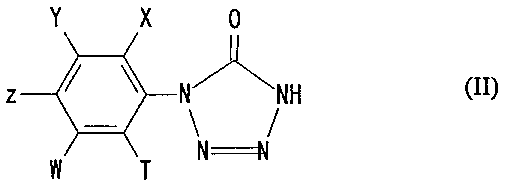 wherein
   X, Y, Z, W and T have the same meanings as stated above,
   are reacted with compounds of the formula (III) 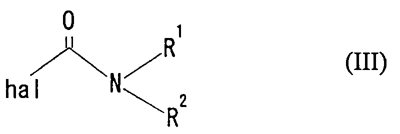 wherein
   R¹ and R² have the same meanings as stated above, and hal is a leaving group such as chlorine and bromine,
in the presence of an inert solvent, and if appropriate, in the presence of an acid binder. The tetrazolinones, according to the invention, exhibit powerful herbicidal properties. Surprisingly, while the tetrazolinones in part fall generically within the scope of the claim of EP-A 0146279, they exhibit a substantially greater herbicidal action than those known from the prior invention.

In the formulae,
halogen includes fluorine, chlorine, bromine and iodine, and preferably represents fluorine, chlorine or bromine,
alkyl and the alkyl parts in alkoxy, alkylthio, alkylsulfonyl and alkylsulfinyl include straight - or branched - chained alkyl, e.g. methyl, ethyl, n-propyl, isopropyl, n-(iso-, sec-, and tert-)-butyl and n-pentyl, and preferably represent methyl or ethyl,
cycloalkyl includes cyclopropyl, cyclobutyl, cyclopentyl, cyclohexyl and cycloheptyl, and preferably represents cyclopropyl, cyclopentyl, cyclohexyl or cycloheptyl, cycloalkenyl includes 1-cyclopentenyl, 2-cyclopentenyl, 3-cyclopentenyl, 1-cyclohexenyl, 2-cyclohexenyl and 3-cyclohexenyl, and preferably represents 1-cyclopentenyl, 2-cyclopentenyl, 1-cyclohexenyl or 2-cyclohexenyl,
a bridged alicyclic grooup includes C₄₋₈, preferably C₄₋₇ bridged polycyclic groups in which 2 or more of alicyclic rings are conbined by co-inclusion of parts of the rings, and preferably represents norbornyl or norbonenyl,
branched alkyl includes isopropyl, isobutyl, sec-butyl, tert-butyl, isopentyl, neopentyl, tert-pentyl, isohexyl and 1,2,2-trimethylpropyl, and preferably represents isobutyl, sec-butyl or 1,2,2-trimethylpropyl, and in the "ring formed together with N-atom, which may be optionally substituted", this ring contains at least one N-atom and optionally at least one further heteroatom such as O or S. Preferably it can be a 5-6 membered monocyclic or condensed polycyclic heterocyclic ring . Then, examples of the nitrogen-containing heterocyclic groups represented by 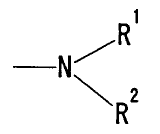
in the formula (I) include piperidino, morpholino, perhydroindol-1-yl, perhydroquinolin-1-yl, 1-indolinyl and the like. In addition, such rings may be substituted in some cases_{,}and examples of the possible substituting groups used in such cases are alkyls such as methyl, ethyl and the like, halogens such as fluorine, chlorine, bromine and the like,among which methyl is preferred.

Among the tetrazolinones according to the invention, of the formula (I), preferred compounds are those
in which
X is hydrogen, fluorine, chlorine, bromine, methyl, methoxy, ethoxy, methylthio, methylsulfonyl, methylsulfinyl, trifluoromethoxy, difluoromethoxy or 2,2,2-trifluoroethoxy,
Y is hydrogen, chlorine, bromine, methyl, ethyl or trifluoromethyl,
Z is hydrogen, fluorine, chlorine, bromine, methoxy, ethoxy or difluoromethoxy,
W is hydrogen, fluorine, chlorine, bromine, methyl, ethyl or trifluoromethyl,
T is hydrogen, fluorine, chlorine, bromine, methyl or ethyl,
R¹ is methyl, ethyl, n-propyl, n-butyl, isobutyl or n-pentyl,
R² is cyclopropyl, C₅₋₇ cycloalkyl which may optionally be substituted by methyl, C₅₋₆ cycloalkenyl, norbornyl, norbornenyl, isobutyl, sec-butyl or 1,2,2-trimethylpropyl, and R¹ and R² may form 2-methylpiperidino, perhydroindol-1-yl or perhydroquinolin-1-yl, together with the nitrogen atom to which they are bonded,
with the proviso that where R² is isobutyl, sec-butyl or 1,2,2-trimethylpropyl, then the total number of the carbon atoms of R¹ and R² is 7 or 8, and
with the exception of the case where
X is chlorine or bromine,
Y, Z and W are bydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8.

Very particularly preferred tetrazolinones of the formula (I) are those in which
X is chlorine, bromine, methyl, methoxy, ethoxy, methylthio, methylsulfonyl or difluoromethoxy,
Y is hydrogen, chlorine, bromine or methyl,
Z is hydrogen, chlorine, bromine or methoxy,
W is hydrogen, chlorine, bromine, methyl or trifluoromethyl,
T is hydrogen, chlorine, bromine or methyl,
R¹ is methyl, ethyl, n-propyl, n-butyl, isobutyl or n-pentyl,
R² is cyclopropyl, C₅₋₆ cycloalkyl which may optionally be substituted by methyl, 1-cyclopentenyl, 2-cyclopentenyl, 1-cyclohexenyl, 2-cyclohexenyl, 2-norbornyl, 5-norbornen-2-yl, isobutyl, sec-butyl or 1,2,2-trimethylpropyl, and
R¹ and R² may form perhydroindol-1-yl or perhydroquinolin-1-yl, together with the nitrogen atom to which they are bonded,
with the proviso that where R² is isobutyl, sec-butyl or 1,2,2-trimethylpropyl, then the total number of the carbon atoms of R¹ and R² is 7 or 8, and
with the exception of the case where
X is chlorine or bromine,
Y, Z and W are hydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8.

The above production method is illustrated by the following reaction scheme in case where, for example, 1-(2-chlorophenyl)-5(4H)-tetrazolinone and N-n-butyl-N-cyclopropyl carbamoyl chloride are used as the starting materials in the above production method. 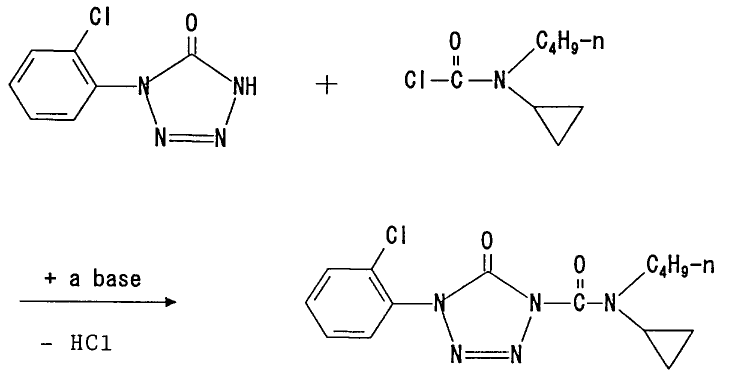
In the above production method, the compounds of formula (II) as the starting materials can be synthesized by a method similar to that described in The Journal of Organic Chemistry, Vol. 45, No. 21 (1980), pp. 5130-5136 or The Journal of American Chemical Society, Vol. 81 (1951), pp. 3076-3079. The typical examples of the compounds of formula (II) are as follows:
1-(2-chlorophenyl)-5(4H)-tetrazolinone,
1-(2-chloro-6-methylphenyl)-5(4H)-tetrazolinone,
1-(2,6-dichlorophenyl)-5(4H)-tetrazolinone,
1-(2-methylphenyl)-5(4H)-tetrazolinone,
1-phenyl-5(4H)-tetrazolinone,
1-(2-methylphenyl)-5(4H)-tetrazolinone,
1-(2-ethylphenyl)-5(4H)-tetrazolinone,
1-(2-methoxyphenyl)-5(4H)-tetrazolinone,
1-(2-ethoxyphenyl)-5(4H)-tetrazolinone,
1-(2-methylthio phenyl)-5(4H)-tetrazolinone,
1-(2-methylsulphonyl phenyl)-5(4H)-tetrazolinone,
1-(2-trifluoromethoxy phenyl)-5(4H)-tetrazolinone,
1-(2-difluoromethoxy phenyl)-5(4H)-tetrazolinone,
1-(2,3-dimethyl phenyl)-5(4H)-tetrazolinone,
1-(2,4-dimethyl phenyl)-5(4H)-tetrazolinone,
1-(2,5-dimethyl phenyl)-5(4H)-tetrazolinone,
1-(2,6-dimethyl phenyl)-5(4H)-tetrazolinone,
1-(3,4-dimethyl phenyl)-5(4H)-tetrazolinone,
1-(3,5-dimethyl phenyl)-5(4H)-tetrazolinone,
1-(2,4,6-trimethyl phenyl)-5(4H)-tetrazolinone,
1-[2-methyl-6-(2,2,2-trifluoroethoxy) phenyl]-5(4H)-tetrazolinone,
1-(2-fluorophenyl)-5(4H)-tetrazolinone,
1-(2,3-dichlorophenyl)-5(4H)-tetrazolinone,
1-(2,4-dichlorophenyl)-5(4H)-tetrazolinone,
1-(2,5-dichlorophenyl)-5(4H)-tetrazolinone,
1-(2-chloro-3-methyl phenyl)-5(4H)-tetrazolinone,
1-(2-chloro-4-methoxyphenyl)-5(4H)-tetrazolinone, and
1-(2-chloro-5-trifluoromethylphenyl)-5(4H)- tetrazolinone.

In the above production method, the compounds of formula (III) as the starting material are those which are well known in the field of organic chemistry and may be exemplified as follows:
N-cyclopropyl-N-n-propyl carbamoyl chloride,
N-cyclopropyl-N-n-butyl carbamoyl chloride,
N-cyclopropyl-N-n-pentyl carbamoyl chloride,
N-2-methylcyclohexyl-N-ethyl carbamoyl chloride,
N-3-methylcyclohexyl-N-ethyl carbamoyl chloride,
N-methylcyclohexyl-N-ethyl carbamoyl chloride,
N-2-methylcyclohexyl-N-n-propyl carbamoyl chloride,
N-2-cyclopentenyl-N-n-propyl carbamoyl chloride,
N-2-cyclohexenyl-N-ethyl carbamoyl chloride,
N-2-norbornyl-N-ethyl carbamoyl chloride,
N-(5-norbornen-2-yl)-N-ethyl carbamoyl chloride,
N-ethyl-N-(1,2,2-trimethylpropyl) carbamoyl chloride,
N-sec-butyl-N-n-propyl carbamoyl chloride,
N,N-diisobutyl carbamoyl chloride,
N-cyclopentyl-N-ethyl carbamoyl chloride,
N-cyclopentyl-N-n-propyl carbamoyl chloride,
N-cyclohexyl-N-ethyl carbamoyl chloride,
N-n-butyl-N-cyclopropyl carbamoyl chloride,
N-methyl-N-(2-methyl cyclohexyl) carbamoyl chloride,
N-ethyl-N-(2-methyl cyclohexyl) carbamoyl chloride,
N-(2-methyl cyclohexyl)-N-n-propyl carbamoyl chloride,
N-methyl-N-(3-methyl cyclohexyl) carbamoyl chloride,
N-ethyl-N-(3-methyl cyclohexyl) carbamoyl chloride,
N-(3-methyl cyclohexyl)-N-n-propyl carbamoyl chloride,
N-methyl-N-(4-methyl cyclohexyl) carbamoyl chloride,
N-ethyl-N-(4-methyl cyclohexyl) carbamoyl chloride,
N-(4-methyl cyclohexyl)-N-n-propyl carbamoyl chloride,
(1-chlorocarbonyl) perhydroindole,
N-cycloheptyl-N-methyl carbamoyl chloride, and
N-cycloheptyl-N-ethyl carbamoyl chloride.

The above production method may be conducted by reacting the compounds of the formula (II) with the compounds of the formula (III) usually in a solvent which is inert to the reaction. Examples of the inert organic solvents useful in the reaction are aliphatic, alicyclic or aromatic hydrocarbons (which may be optionally chlorinated) such as pentane, hexane, cyclohexane, petroleum ether, ligroin, benzene, toluene, xylene, dichloromethane, chloroform, carbon tetrachloride, 1,2-dichloroethane, chlorobenzene and dichlorobenzene; ethers such as diethyl ether, methylethyl ether, diisopropyl ether, dibutyl ether, dioxane, dimethoxy ethane (DME), tetrahydrofuran (THF) and diethyleneglycol dimethyl ether (DGM); nitriles such as acetonitrile and propionitrile; acid amides such as dimethyl formamide (DMF), dimethyl acetoamide (DMA), N-methyl pyrrolidone, 1,3-dimethyl-2-imidazolidinone and hexamethyl phosphoric triamide (HMPA); sulphones and sulphoxides such as dimethyl sulphoxide (DMSO) and sulfolan; and bases such as pyridine.

The above reaction can be carried out in the presence of an acid binding agent and the followings are to be mentioned as examples of the useful acid binding agents: as inorganic bases, carbonates and bicarbonates of alkali metals such as sodium hydrogencarbonate, potassium hydrogencarbonate, sodium carbonate, potassium carbonate, etc., and, as organic bases, tertiary amines, dialkylamino anilines and pyridines such as triethylamine, 1,1,4,4-tetramethyl ethylenediamine (TMEDA), N,N-dimethyl aniline, N,N-diethyl aniline, pyridine, 4-dimethylamino pyridine (DMAP), 1,4-diazabicyclo[2,2,2]octane (DABCO) and 1,8-diazabicyclo[5,4,0]undec-7-ene (DBU) and the like.

Furthermore, 4-dimethylamino pyridine can be used as the catalyst and/or the acid binding agent in order to selectively synthesize the desired compounds.

The above reaction can be practiced at a temperature within a broad range but generally it is preferable to practice it at a temperature within the range of about -30 to about 200°C, in particular, about -20 to about 130°C. The reaction should preferably be practiced under atmospheric pressure but it may be optionally practiced under an elevated or reduced pressure.

The compounds of formula (I) according to the invention can be obtained, for instance, by reacting one mole of the compound of formula (II) with 1 to 1.2 moles of the compound of formula (III) in the presence of 1 to 1.2 moles of the acid binding agent and 4-dimethylamino pyridine.

The compounds of formula (I) thus obtained can be isolated and purified by a method such as crystallization, chromatography and the like.

The compounds of formula (I) according to the invention have, as shown in the test examples afterward, excellent herbicidal activity so that they can be used as a herbicide for controlling weeds. The term "weeds" means all plants which grow in undesired loci.

The compounds according to the invention act as non-selective or selective herbicides in dependence on the concentration used.

The compounds according to the invention can be used, for example, as selective herbicides in connection with the following weeds and the cultivated plants.

Dicotyledon weeds of the genera: Sinapis, Lepidium, Galium, Stellaria, Chenopodium, Urtica, Senecio, Amaranthus, Portulaca, Xanthium, Ipomoea, Polygonum, Ambrosia, Cirsium, Sonchus, Solanum, Rorippa, Lamium, Veronica, Datura, Viola, Galeopsis, Papaver, Centaurea, Galinsoga, Rotala, Lindernia, etc.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita, etc.

Monocotyledon weeds of the genera: Echinochloa, Setaria, Panicum, Digitaria, Phleum, Poa, Festuca, Eleusine, Lolium, Bromus, Avena, Cyperus, Sorghum, Agropyron, Monochoria, Fimbristylis, Saggitaria, Eleocharis, Scirpus, Paspalum, Ischaemum, Agrostis, Alopecurus, Cynodon, etc.

Monocotyledon cultures of the genera: Oryza, Zea, Triticum, Hordeum, Avena, Secale, Sorghum, Panicum, Saccharum, Ananas, Asparagus, Allium, etc.

However, the use of the compounds according to the invention is in no way restricted to the above genera, but also extends in the same manner to other plants. Further, the inventive compounds are suitable, depending on the concentration, for the total combating of weeds, for example on industrial terrain, rail tracks, and on paths and squares with or without tree plantings.

Equally, the compounds of the invention can be employed for combating weeds in perennial cultures, for example afforestations, decorative tree plantations, orchards, vineyards, citrus groves, nuts orchards, banana plantations, coffee plantations, tea plantations, rubber plantations, oil palm plantations, cocoa plantations, soft fruit plantations and hopfields, and for the selective combating of weeds in annual cultures.

The active compounds of the invention can be converted to the customary formulations, such as solutions, emulsions, wettable powders, suspensions, powders, soluble powders, dusting agents, granules, suspension-emulsion concentrates, very fine capsules in polymeric substances, natural and synthetic materials impregnated with active compound, etc.

Those formulations are produced in the manner known per se, for example, by mixing the active compounds with extenders, that is liquid solvents and/or solid carriers, optionally with the use of surface-active agents, that is emulsifying agents and/or dispersing agents and/or foam-forming agents.

In the case of the use of water as an extender, organic solvents can be used as auxiliary solvents. As liquid solvents, there are suitable in the main: aromatic hydrocarbons, such as xylene, toluene or alkyl naphthalenes; chlorinated aromatic hydrocarbons and chlorinated aliphatic hydrocarbons; such as chlorobenzenes, chloroethylenes or methylene chloride; aliphatic hydrocarbons, such as cyclohexane or paraffins, for example petroleum fractions, mineral and vegetable oils, alcohols, such as butanol or glycol as well as their ethers and esters, ketones, such as acetone, methyl ethyl ketone, methyl isobutyl ketone or cyclohexanone; strongly polar solvents, such as dimethylformamide and dimethylsulphoxide; as well as water.

As solid carriers there are suitable: for example, ammonium salts and ground natural minerals, such as kaolins, clays, talc, chalk, quartz, attapulgite, montmorillonite or diatomaceous earth, and ground synthetic minerals, such as highly disperse silicic acid, alumina and silicates. As solid carriers for granules there are suitable: for example, crushed and fractionated natural rocks such as calcite, marble, pumice, sepiolite and dolomite, as well as synthetic granules of inorganic and organic meals, and granules of organic material such as sawdust, coconut shells, maize and tobacco stalks.

As emulsifying and/or foam-forming agents there are suitable: for example non-ionic and anionic emulsifiers, such as polyoxyethylene-fatty acid esters, polyoxyethylene-fatty alcohol ethers, for example alkylaryl polyglycol ethers, alkylsulphonates, alkylsulphates, arylsulphonates as well as albumin hydrolysation products.

As dispersing agents there are suitable: for example lignin-sulphite waste liquors and methylcellulose.

Adhesives may also be used optionally in formulations such as powders, granules, natural and synthetic materials impregnated with active compound or emulsions, and the followings are to be mentioned as examples of such adhesives: for example carboxymethylcellulose and natural and synthetic polymers such as gum arabic, polyvinyl alcohol and polyvinyl acetate, as well as natural phospholipids, such as cephalins and lecithins, synthetic phospholipids. As further additives there are mineral and vegetable oils used.

It is possible to use colorants such as inorganic pigments, for example iron oxide, titanium oxide and Prussian Blue, and organic dyestuffs, such as alizarin dyestuffs, azo dyestuffs and metal phthalocyanine dyestuffs, and trace nutrients such as salts of metals, for example iron, manganese, boron, copper, molybdenum and zinc.

The formulations in general contain between 0.1 and 95 per cent by weight preferably between 0.5 and 90% by weight of the active compound.

The active compounds of the invention can be used for controlling of weeds as they are or in a form of such formulations and can be mixed with any of known herbicides. The mixture may be either prepared in advance in the form of a final formulation or prepared by tank-mixing immediately before use.

It is possible to mix the active compounds of the invention with a chemical injury-mitigating agent and the applicability as a selective herbicide can be more broadened by this mixing.

The chemical injury-mitigating agent may be exemplified by 1-(α,α-dimethyl benzyl)-3-p-tolyl urea.

The active compounds of the invention may be applied by any of conventional methods such as watering, atomizing, powder spreading or granule scattering.

The active compounds of the invention may be applied at any stage of preemergence or postemergence. Also, they can be incorporated into the soil before sowing.

The application amount of the active compound is not strictly limited and may be varied within a wide range depending on the nature of desired effect, the kind of target plant(s) as the object, the location of application, the time of application and the like but, as a tentative measure, the amount can be exemplified by about 0.001 kg/ha to about 10 kg/ha, preferably about 0.01 kg/ha - about 5 kg/ha of the active compound.

Then, the following examples illustrate the production and uses of the compounds of the invention, but they should not be regarded as limiting the invention in any way. The term "part(s)" therein means "part(s) by weight" unless otherwise noted.

### Examples

### Synthesis Example 1

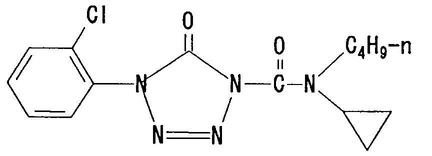

4-Dimethylaminopyridine (0.79 g) is added to a solution of 1-(2-chlorophenyl)-5(4H)-tetrazolinone (0.98 g) and N-n-butyl-N-cyclopropylcarbamoyl chloride (1.05 g) in acetonitrile (30 mℓ) and is reacted at 50-55°C for 5 hours.

After distilling off of the solvent under a reduced pressure, ethyl acetate (40 mℓ) is added to the residue and washed with 10% hydrochloric acid (10 mℓ) and then with water (10 mℓ) and dried over anhydrous magnesium sulfate. The desired 1-(2-chlorophenyl)-4-(N-n-butyl-N-cyclopropyl carbamoyl)-5(4H)-tetrazolinone (1.13 g) is obtained by crystallization from hexane after distilling off of the solvent under a reduced pressure. m.p. 93.5-95.5°C.

### Synthesis Example 2

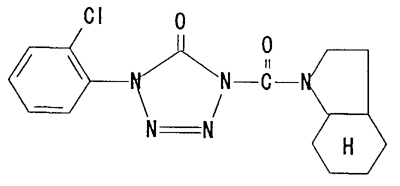

4-Dimethylaminopyridine (1.59 g) is added to a solution of 1-(2-chlorophenyl)-5(4H)-tetrazolinone (1.97 g) and (1-chlorocarbonyl) perhydroindole (2.25 g) in toluene (40 mℓ) and is reacted at 50-55°C for 12 hours. The reaction solution is washed with 10 % hydrochloric acid (20 mℓ) and then with water (20 mℓ) followed by drying over anhydrous magnesium sulphate. The solvent is distilled off under a reduced pressure and the residue is crystallized from hexane to obtain the desired 1-(2-chlorophenyl)-4-(perhydroindol-1-yl) carbonyl-5(4H)-tetrazolinone (2.80 g). mp. 130-131.5°C

### Synthesis Example 3

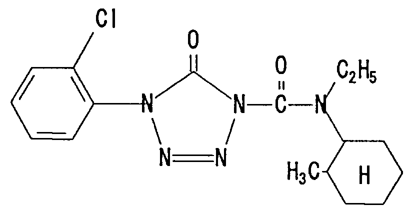

1-(2-Chlorophenyl)-5(4H)-tetrazolinone (2 g), 4-dimethylaminopyridine (1.49 g) and N-ethyl-N-2-methylcyclohexyl carbamoyl chloride (2.49 g) are dissolved in acetonitrile (30 mℓ) and are heated for 6 hours with refluxing. After distilling off of the solvent under a reduced pressure, chloroform (30 mℓ) is added to the residue and washed with water (20 mℓ x 2). After drying of the chloroform layer over anhydrous sodium sulphate, the solvent is distilled off under a reduced pressure and the residue is subjected to flash column chromatography (eluent: hexane/ethyl acetate = 3/1) to obtain the desired 1-(2-chlorophenyl)- 4-(N-ethyl-N-2-methylcyclohexyl carbamoyl)-5(4H)-tetrazolinone (1.57 g).
n_{D}²⁰ 1.5302

### Synthesis Example 4

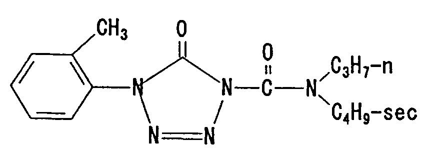

1-(2-methylphenyl)-5(4H)-tetrazolinone (2 g), 4-dimethylamino pyridine (1.66 g) and N-sec-butyl-N-n-propyl carbamoyl chloride (2.42 g) are dissolved in acetonitrile (30 mℓ) and heated for 6 hours with refluxing. After distilling off of the solvent under a reduced pressure, chloroform (30 mℓ) is added to the residue and washed with water (20 mℓ x 2). After drying, the chloroform layer is dried over anhydrous sodium sulphate, the solvent is distilled off under a reduced pressure and the residue is subjected to flash column chromatography (eluent: hexane/ethyl acetate = 5/1) to give the desired 1-(2-methylphenyl)-4-(N-sec-butyl- N-n-propyl carbamoyl)-5(4H)-tetrazolinone (1.88 g).
n_{D}²⁰ 1.5211

### Synthesis Example 5

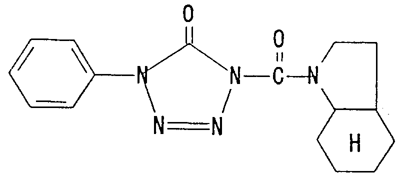

4-Dimethylamino pyridine (0.48 g) is added to a solution of 1-phenyl-5(4H)-tetrazolinone (0.58 g) and (1-chlorocarbonyl) perhydroindole (0.56 g) in toluene (25 mℓ) and reacted at 50-55°C for 12 hours. After washing with 10 % hydrochloric acid (10 mℓ), with water (10 mℓ) and then with a saturated sodium hydrogen carbonate solution (10 mℓ), the reaction solution is dried over anhydrous magnesium sulphate. By distilling off of the solvent under a reduced pressure and
purification of the residue through silica gel column chromatography, the desired 1-phenyl-4-(perhydroindol-1-yl) carbonyl 5(4H)-tetrazolinone (0.77 g) is obtained.
n_{D}²⁰ : 1.5746

### Synthesis Example 6

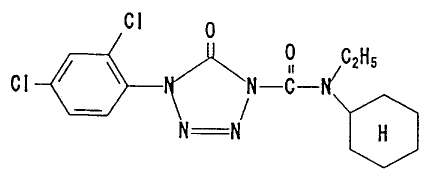

1-(2,4-Dichlorophenyl)-5(4H)-tetrazolinone (2 g), 4-dimethylamino pyridine (1.26 g) and N-cyclohexyl-N-ethyl carbamoyl chloride (1.97 g) are dissolved in acetonitrile (30 mℓ) and heated for 6 hours with refluxing. After distilling off of the solvent under a reduced pressure, chloroform (30 mℓ) is added to the residue and washed with water (20 mℓ x 2). After drying of the chloroform layer over anhydrous sodium sulfate, the solvent is distilled off under a reduced pressure and the residue is subjected to flash column chromatography (eluent: hexane/ethyl acetate = 4/1) to obtain the desired 1-(2,4-dichlorophenyl)-4-(N-cyclohexyl-N-ethyl carbamoyl)-5(4H)-tetrazolinone (1.59 g).
n_{D}²⁰ 1.5463
Further compounds obtainable by the above-mentioned procedure are shown in the following Tables 1 to 4, together with the compounds obtained in the foregoing Synthesis Examples.

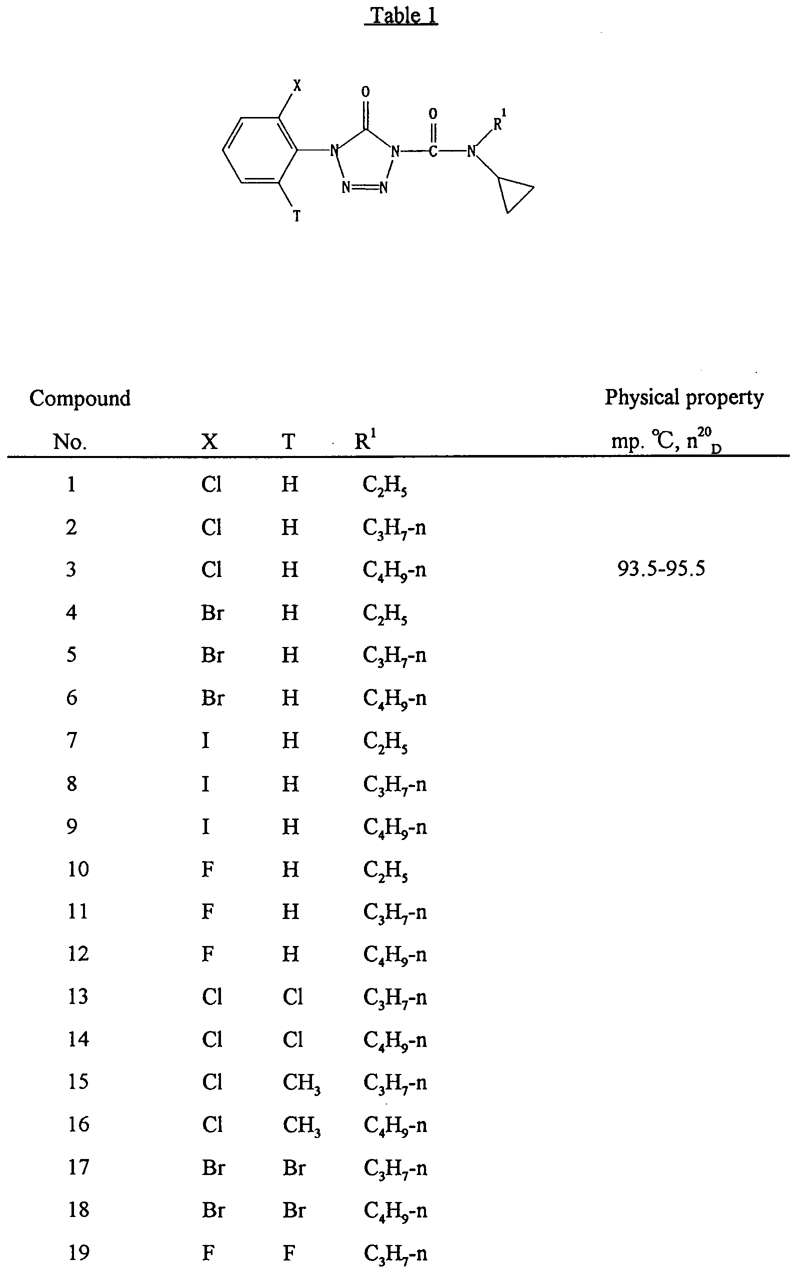

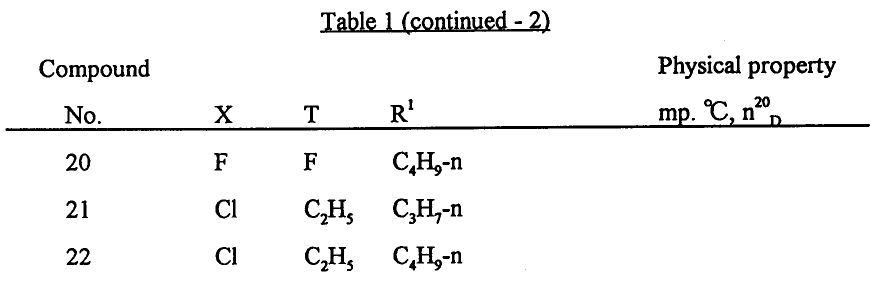

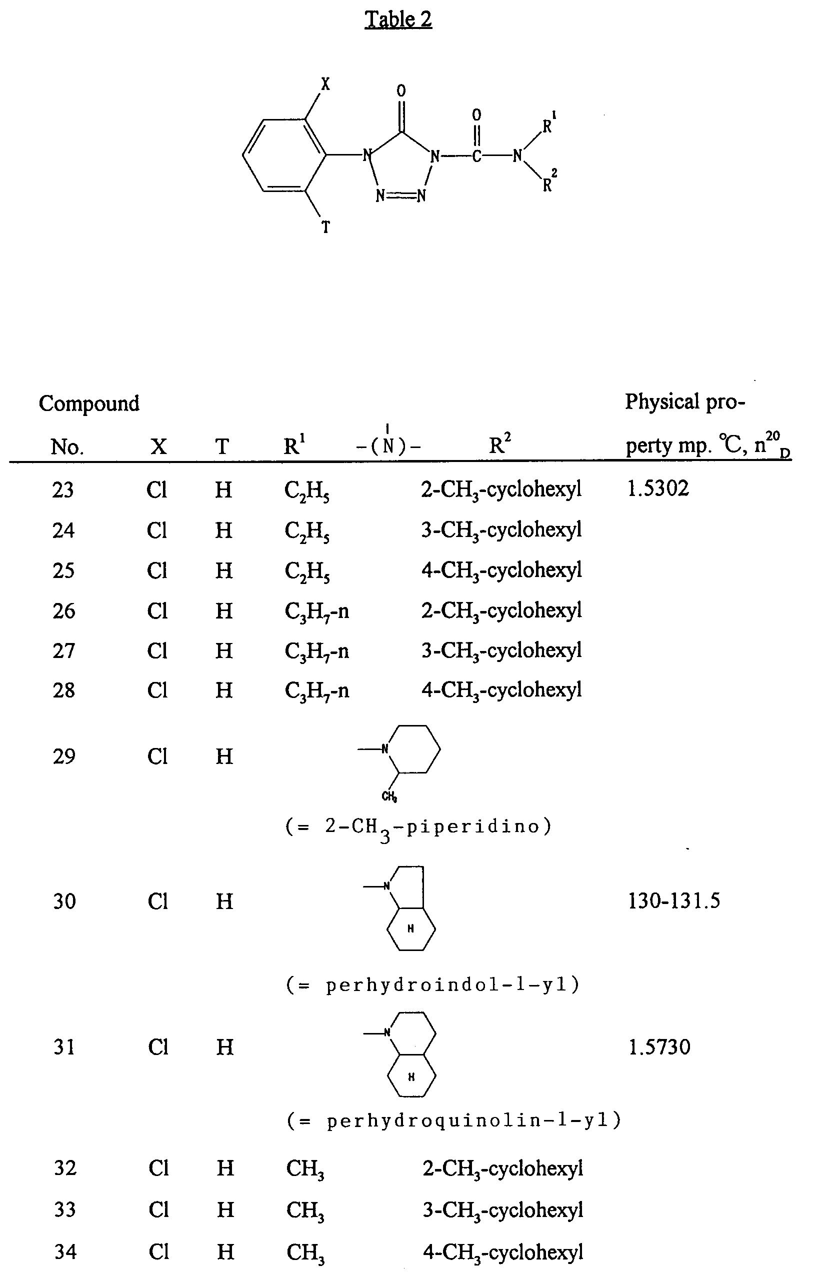

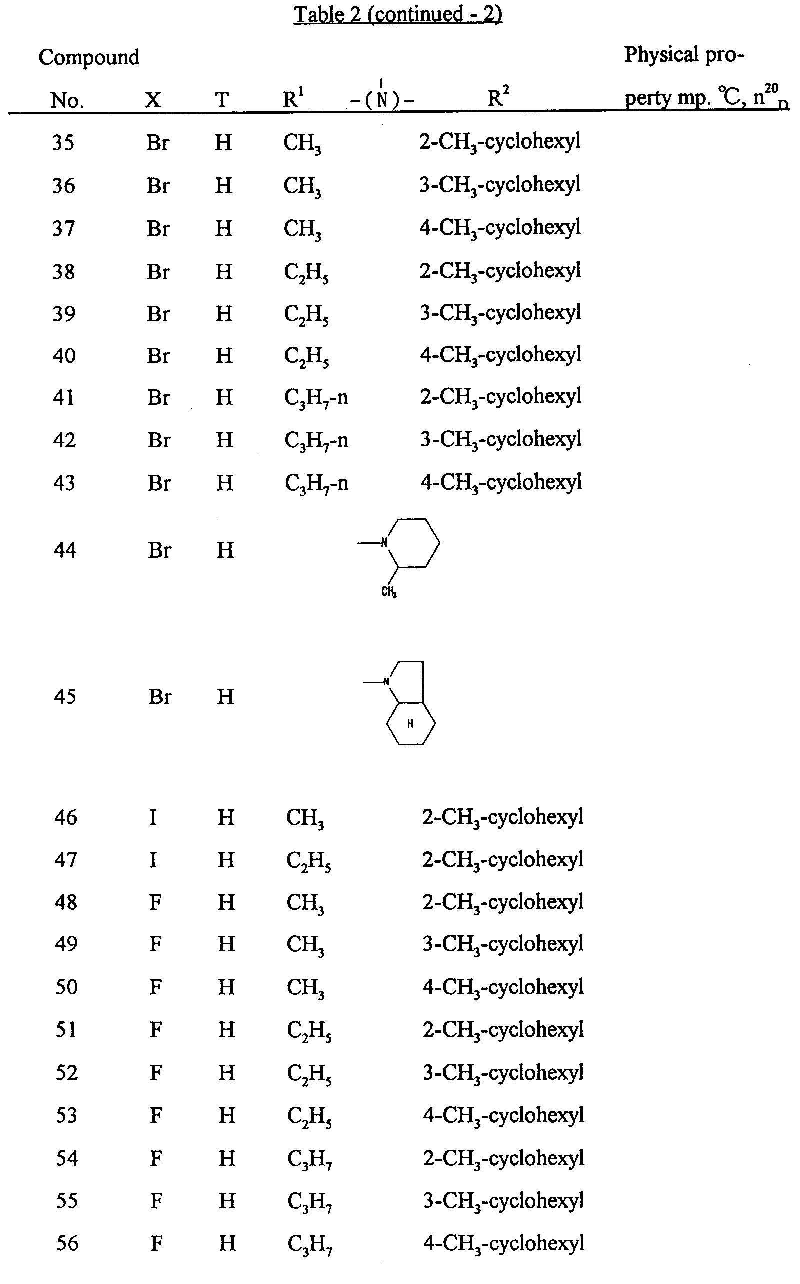

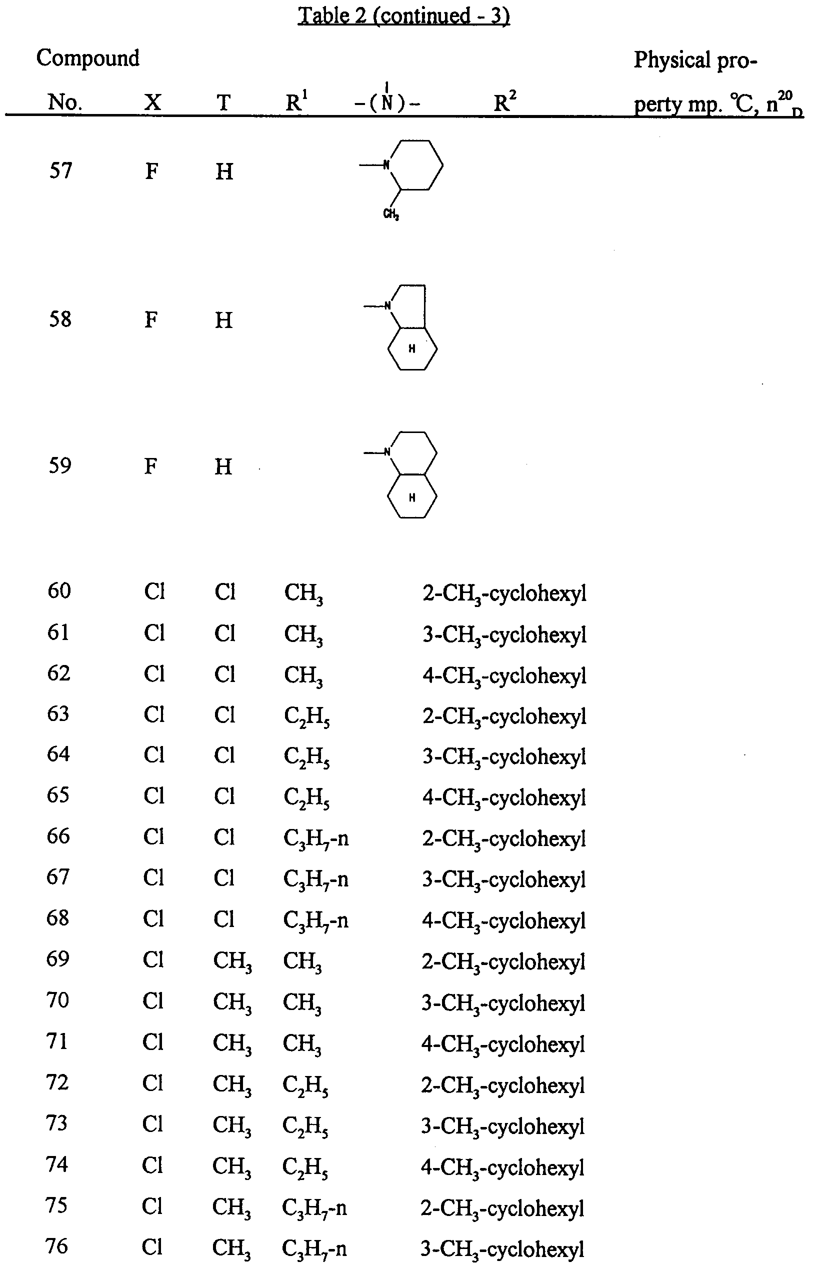

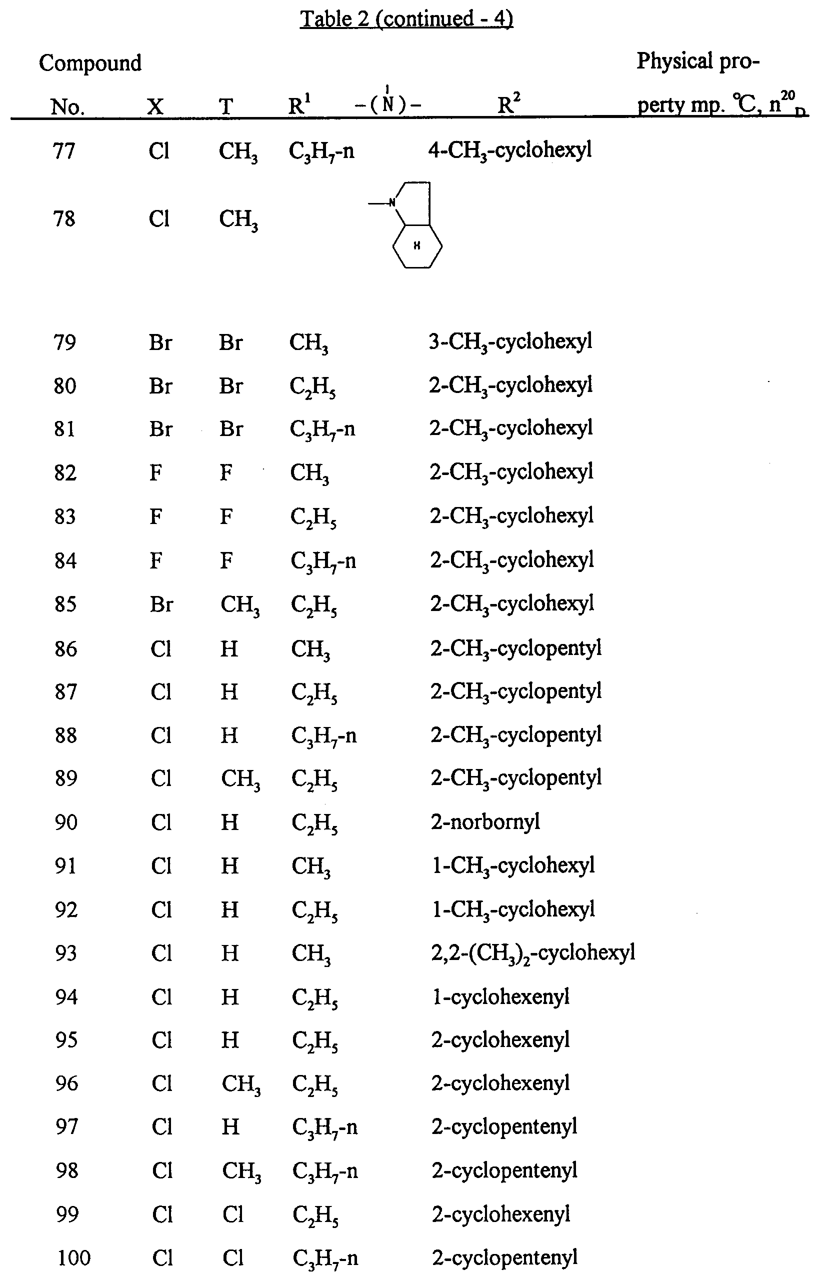

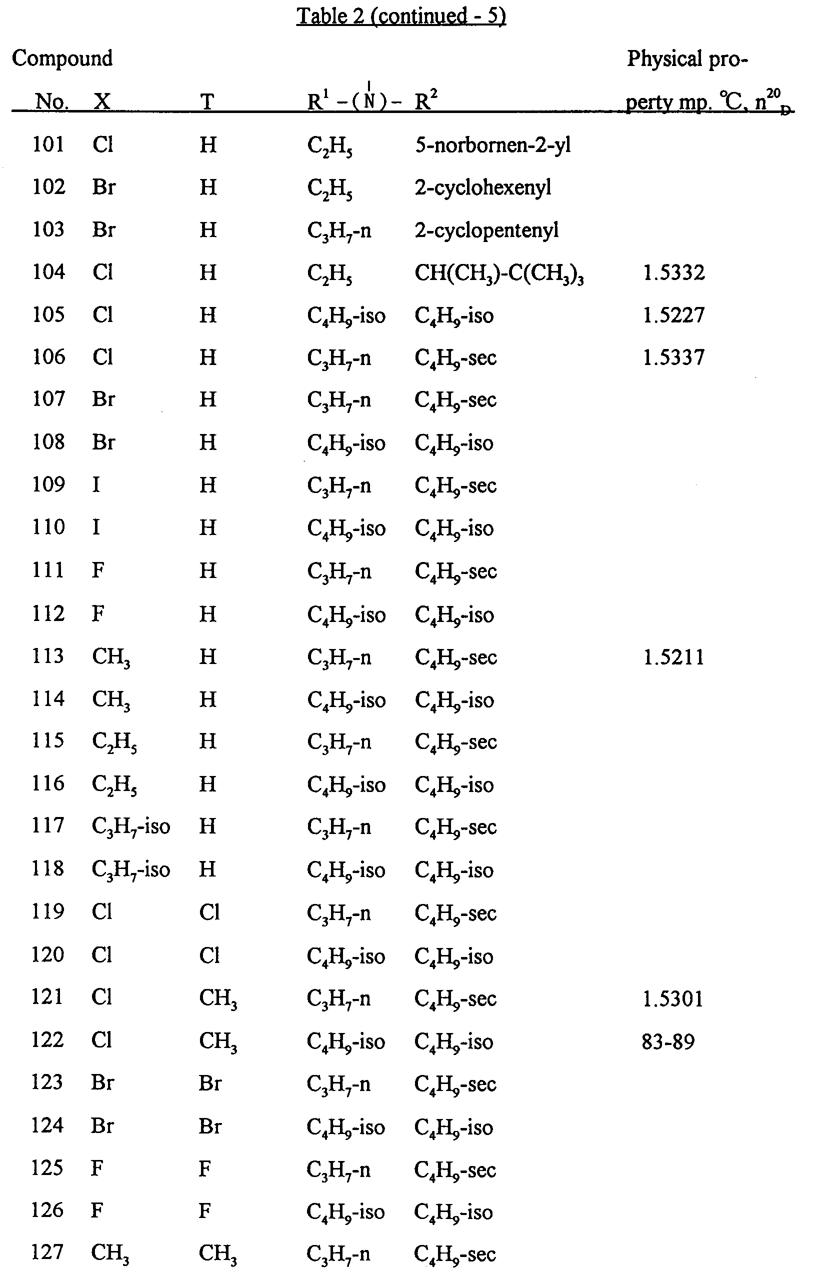

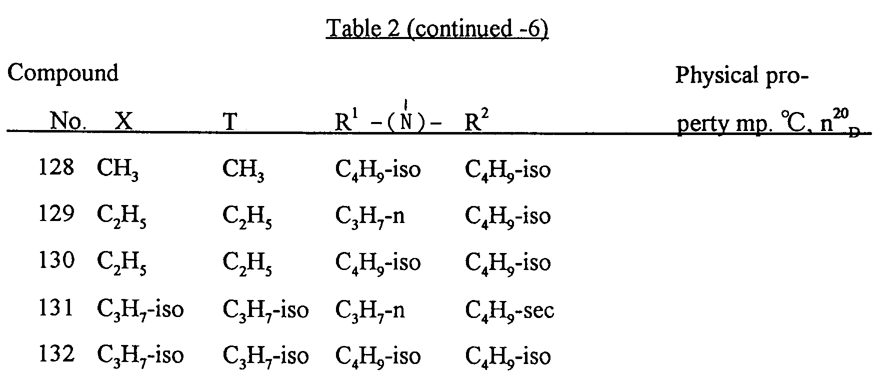

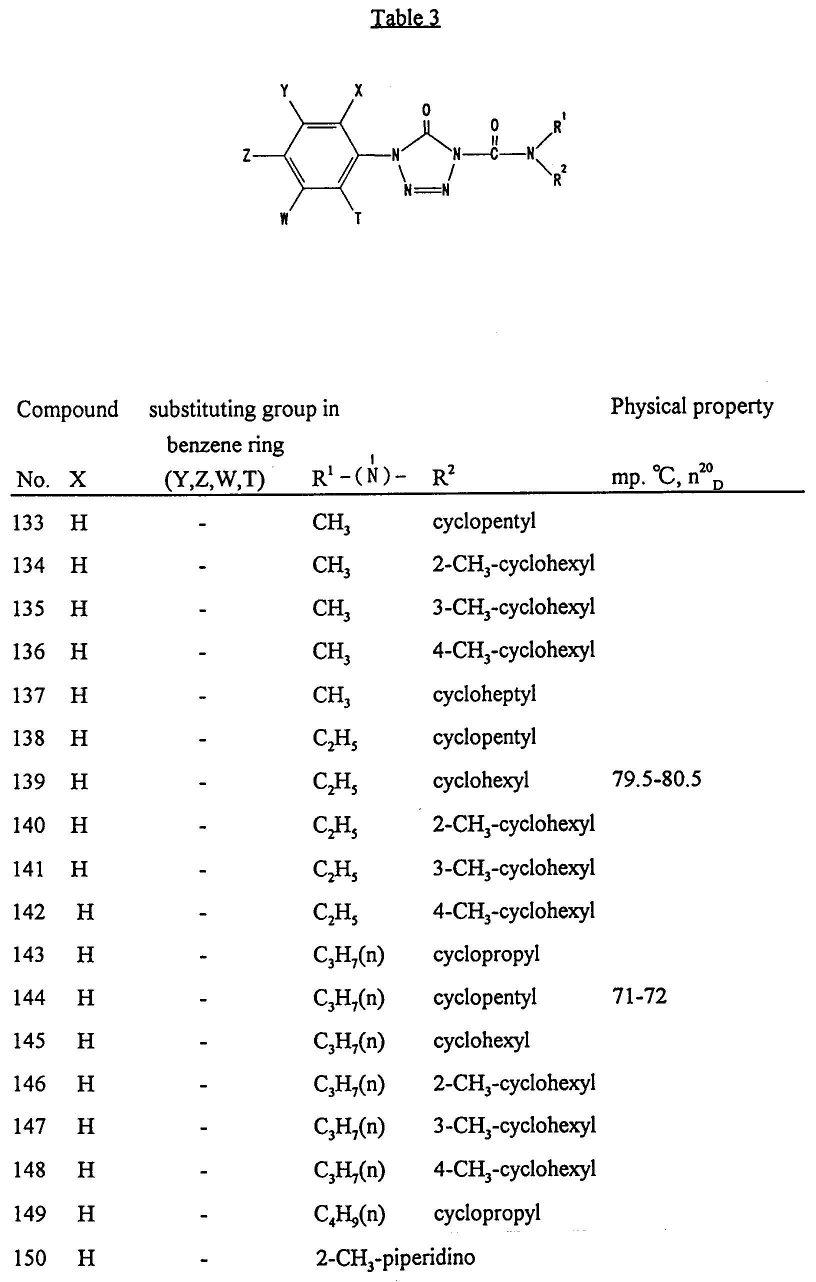

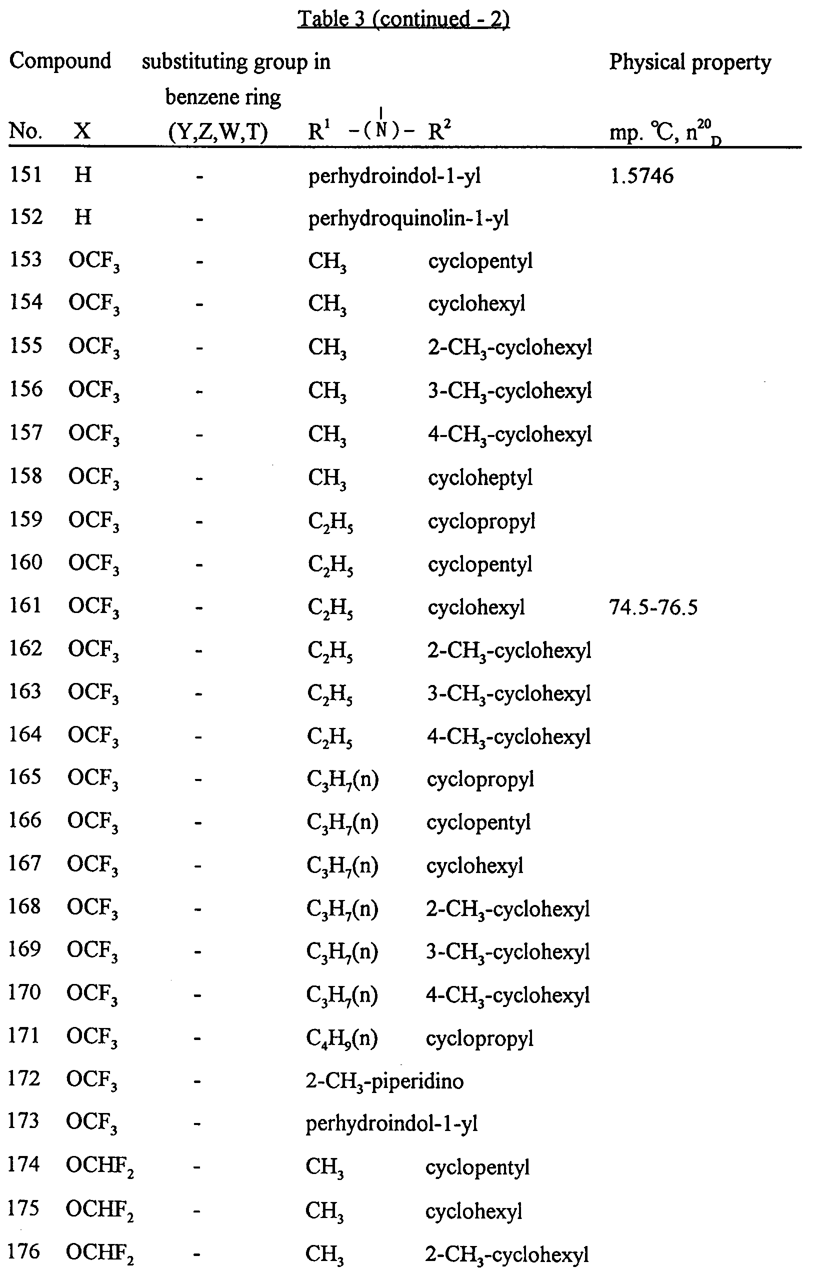

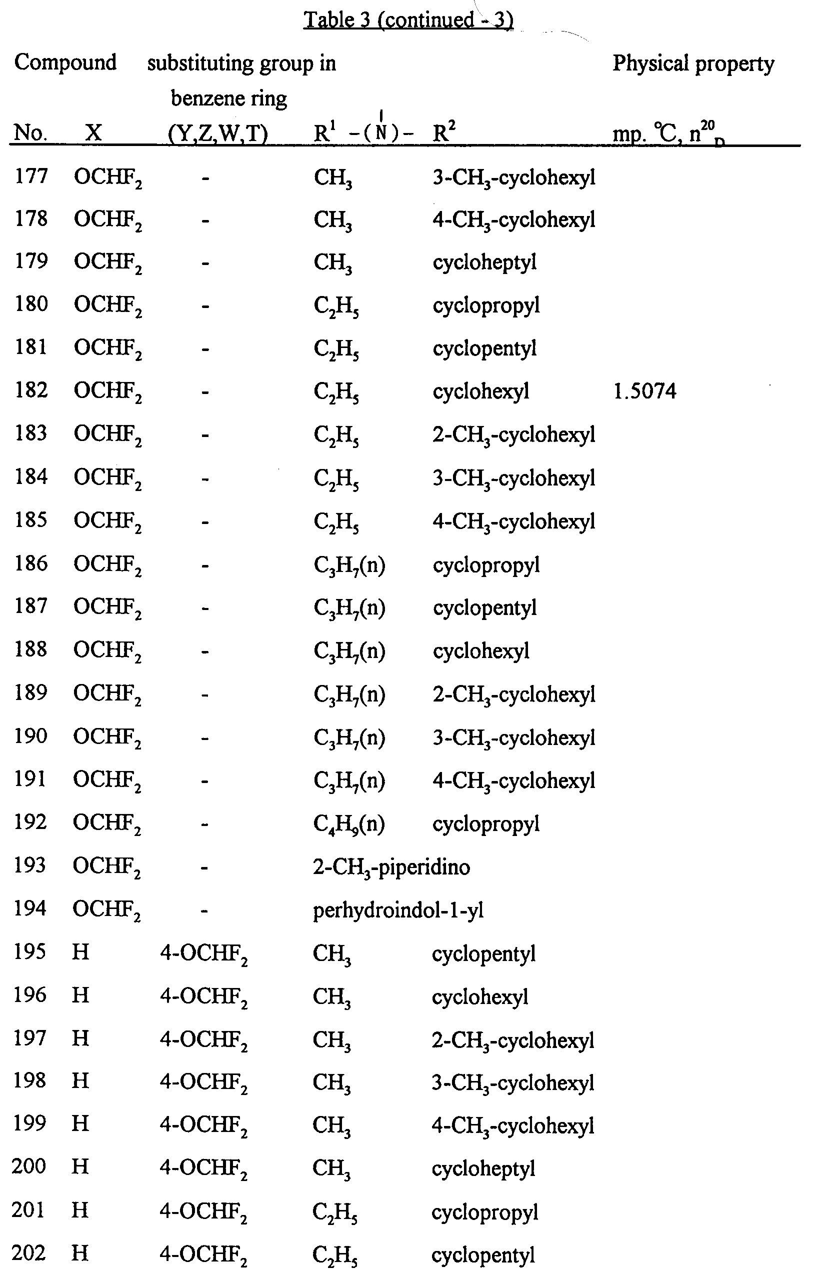

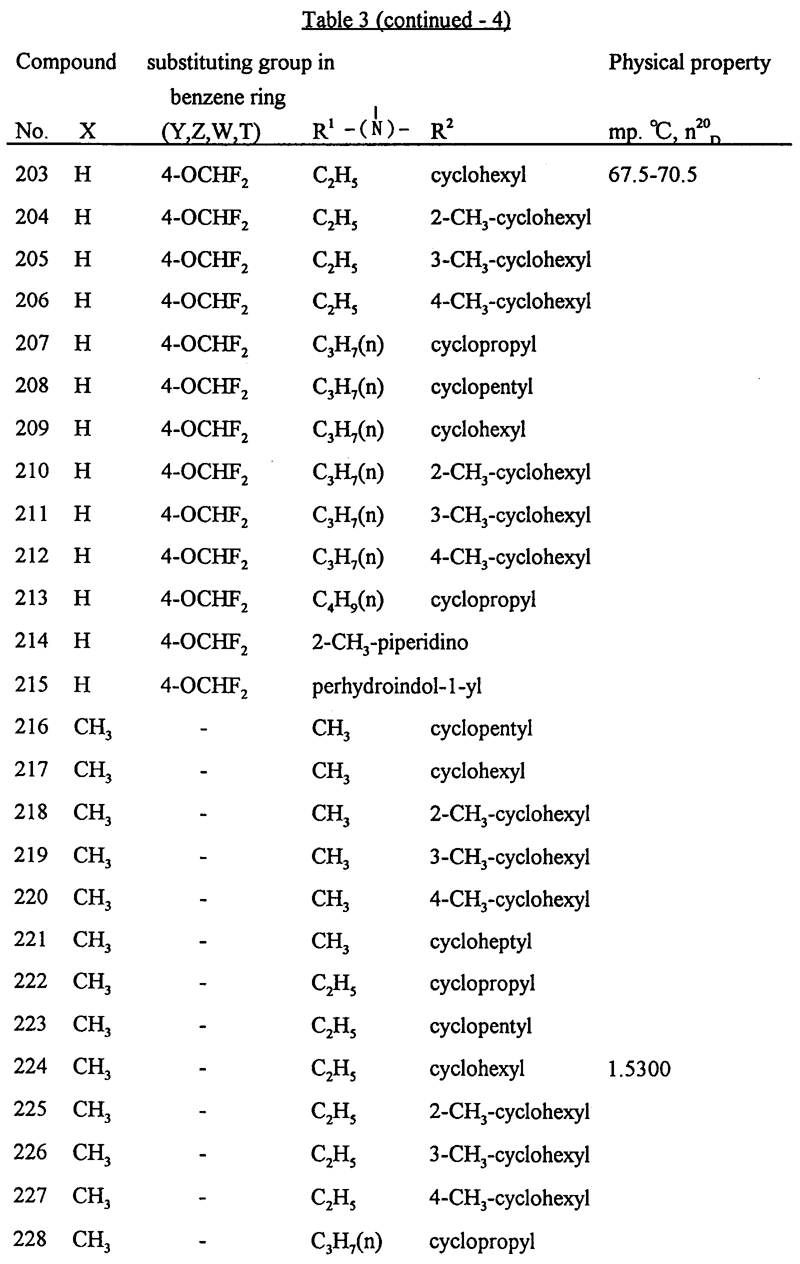

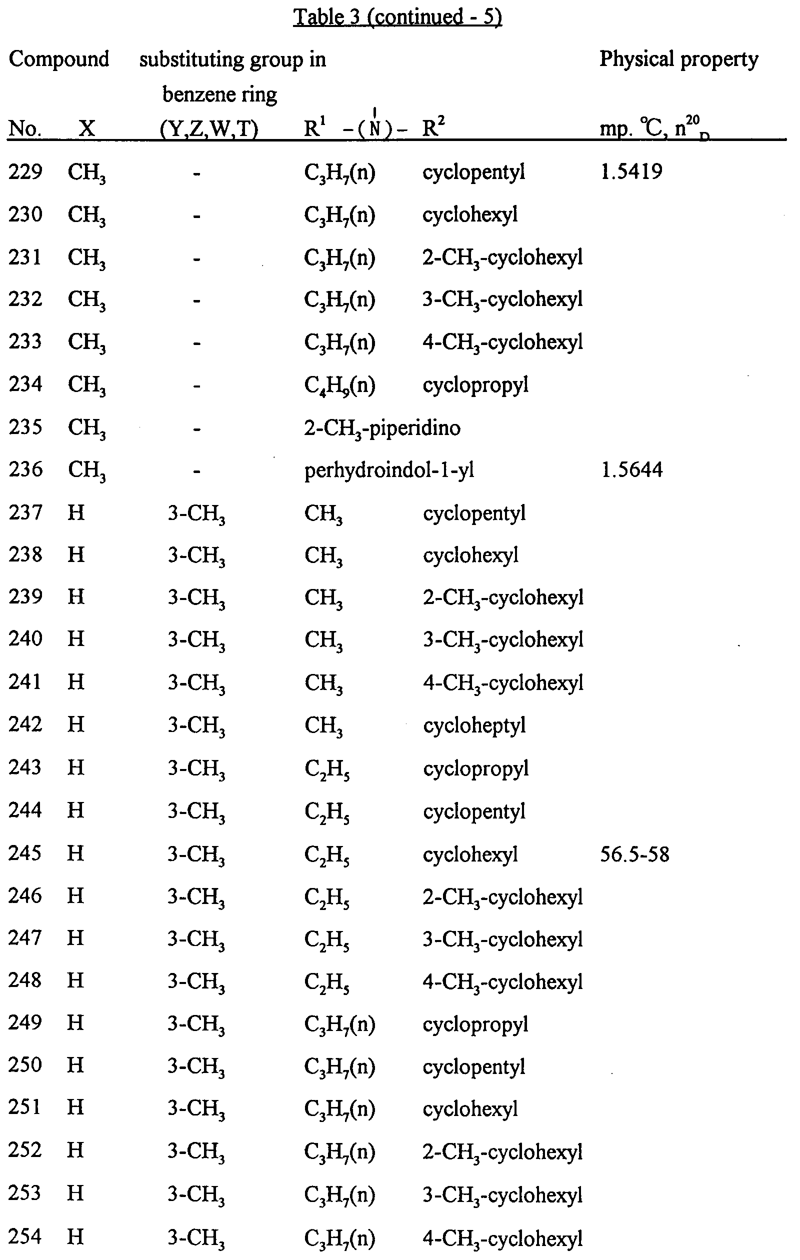

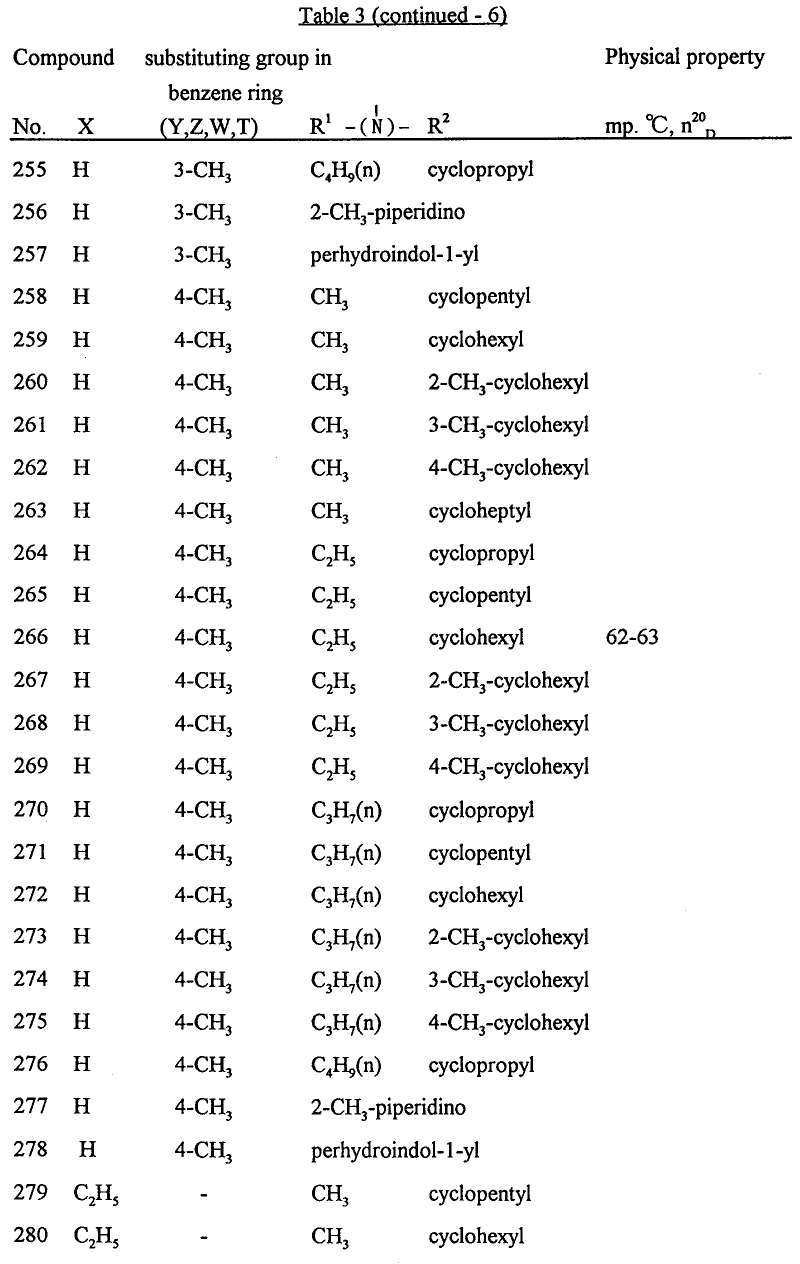

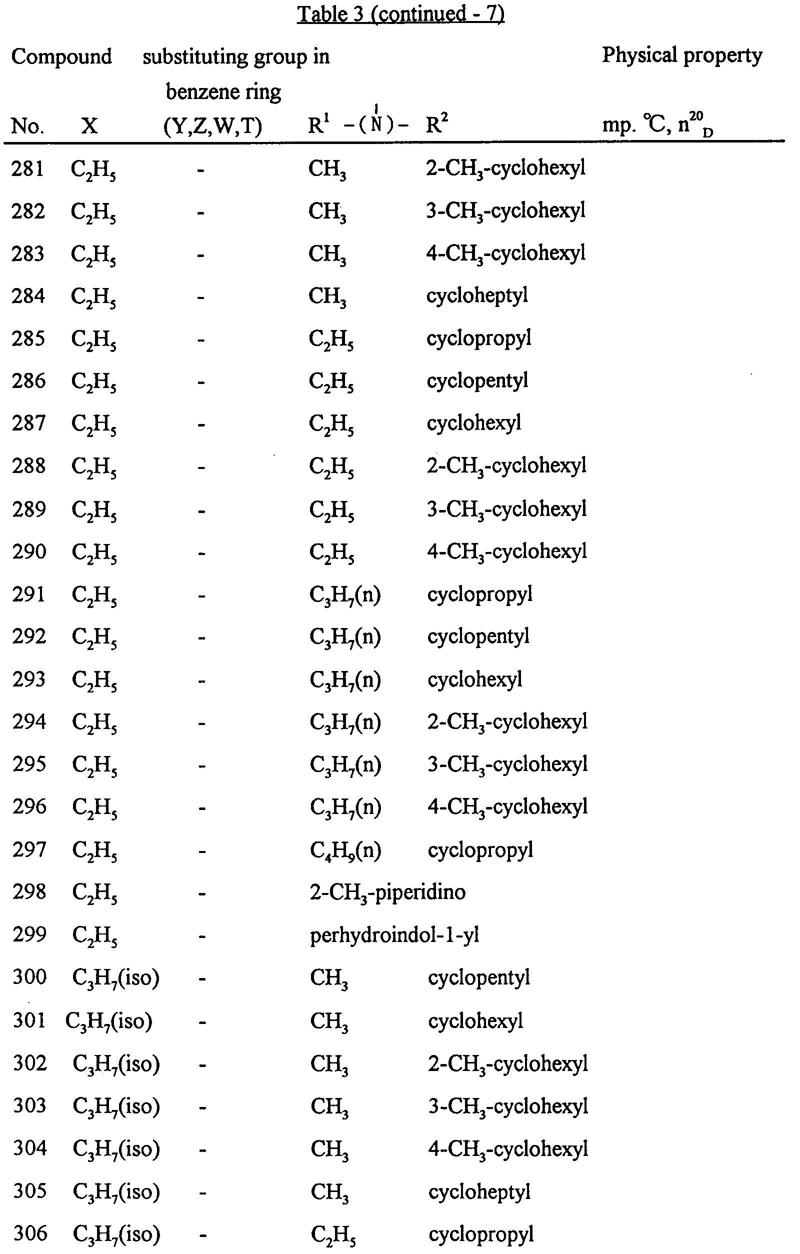

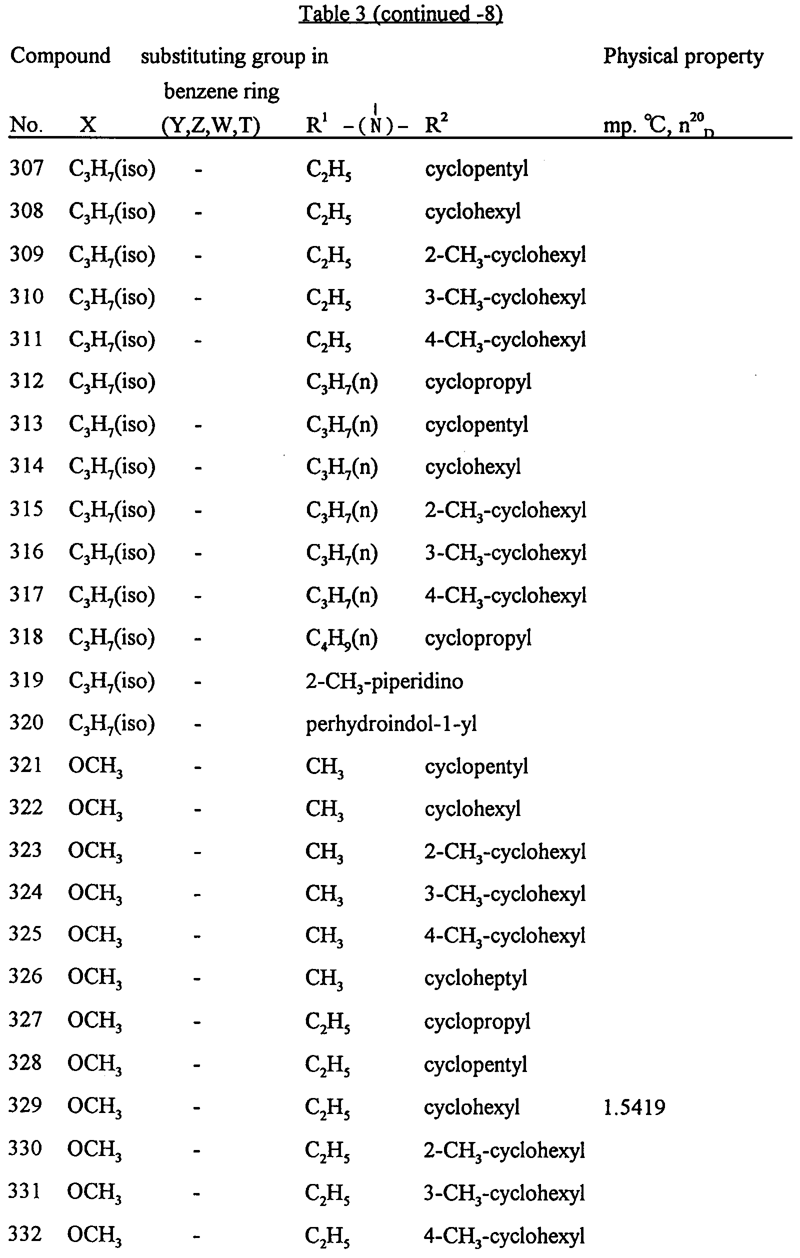

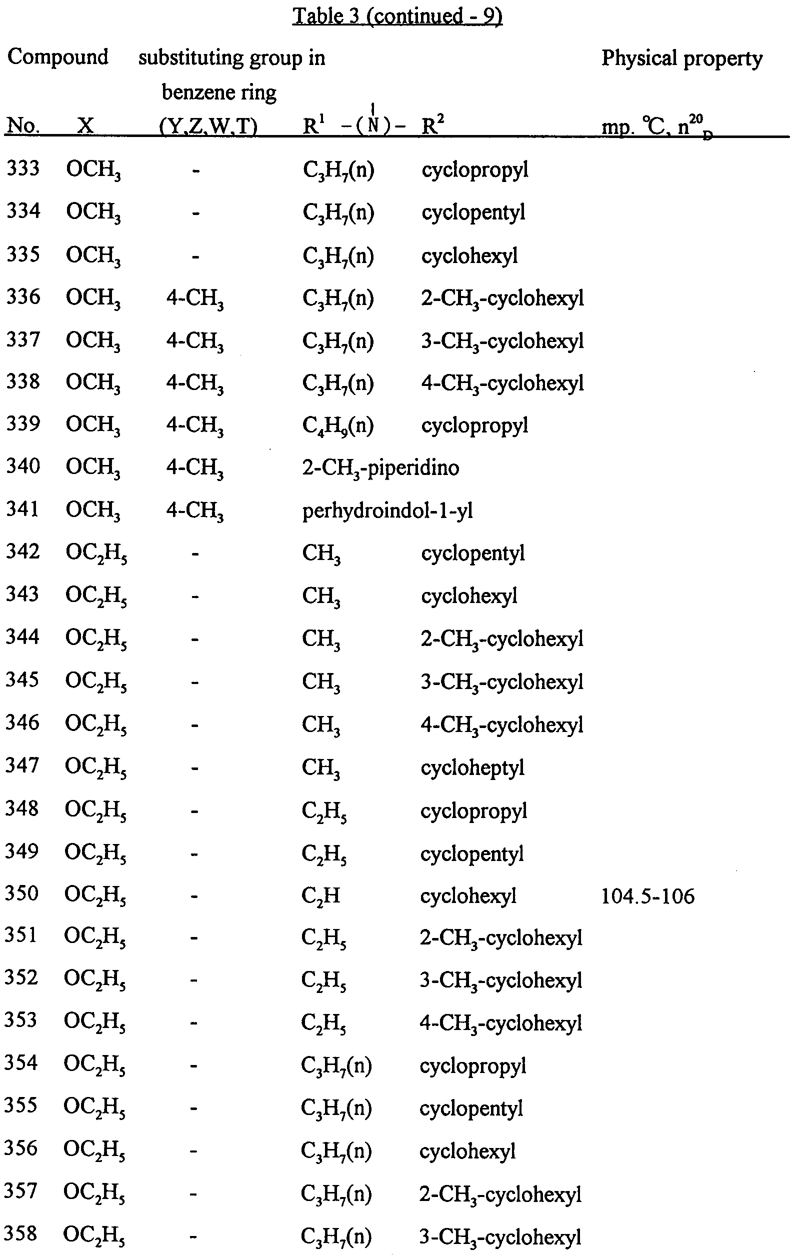

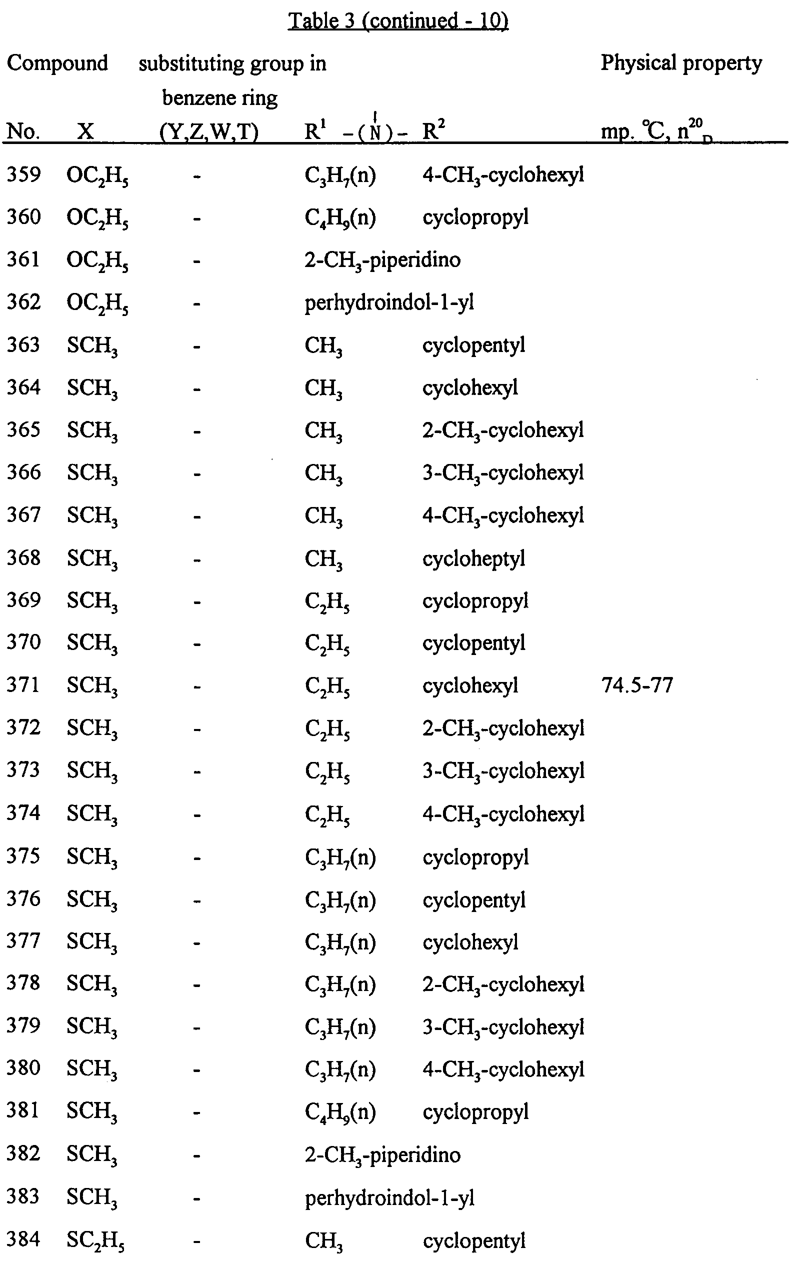

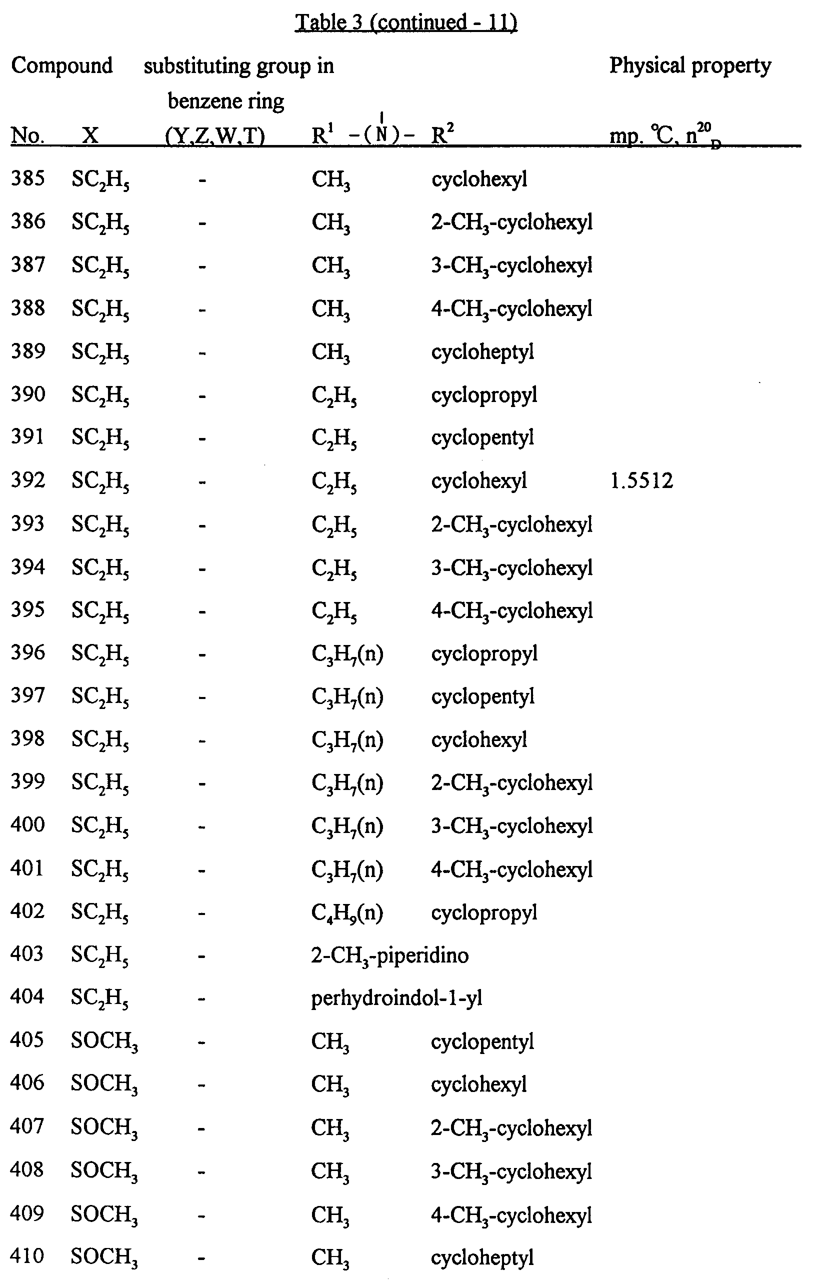

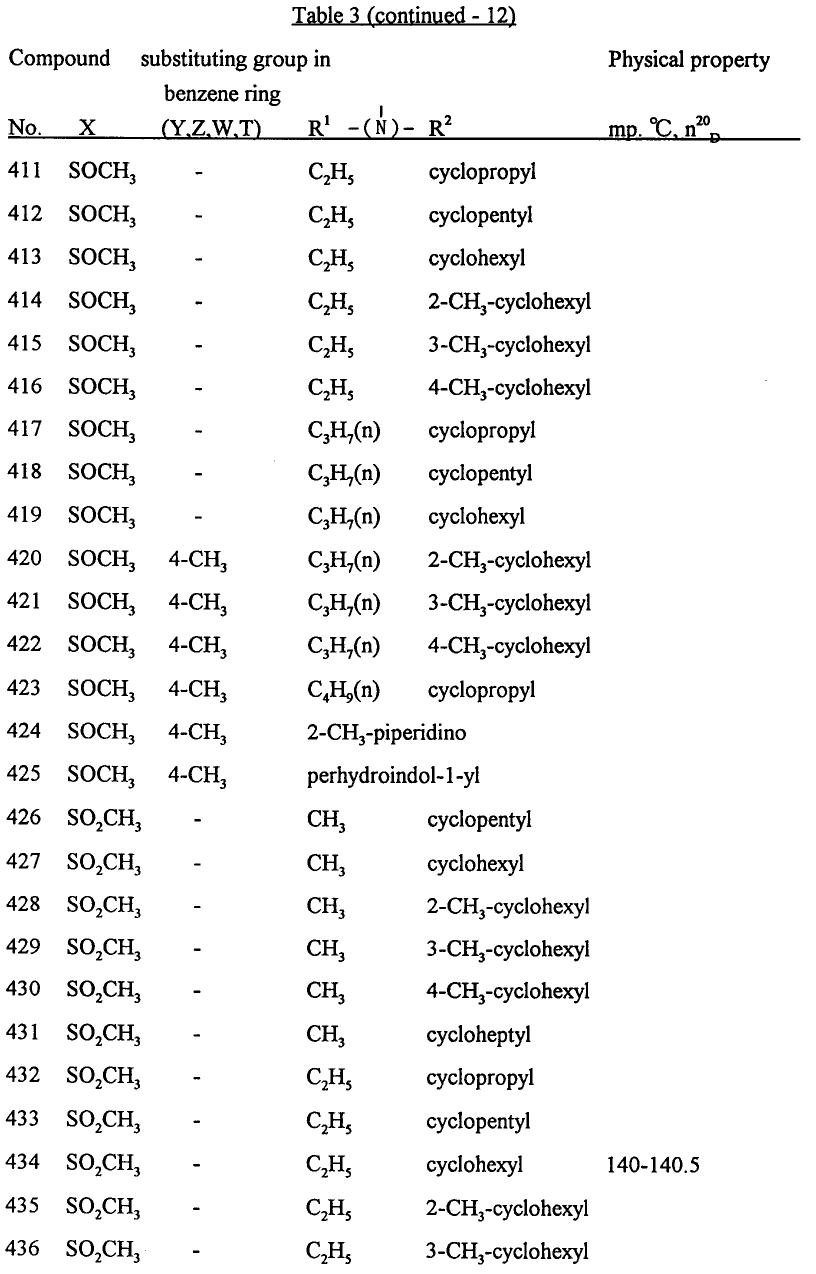

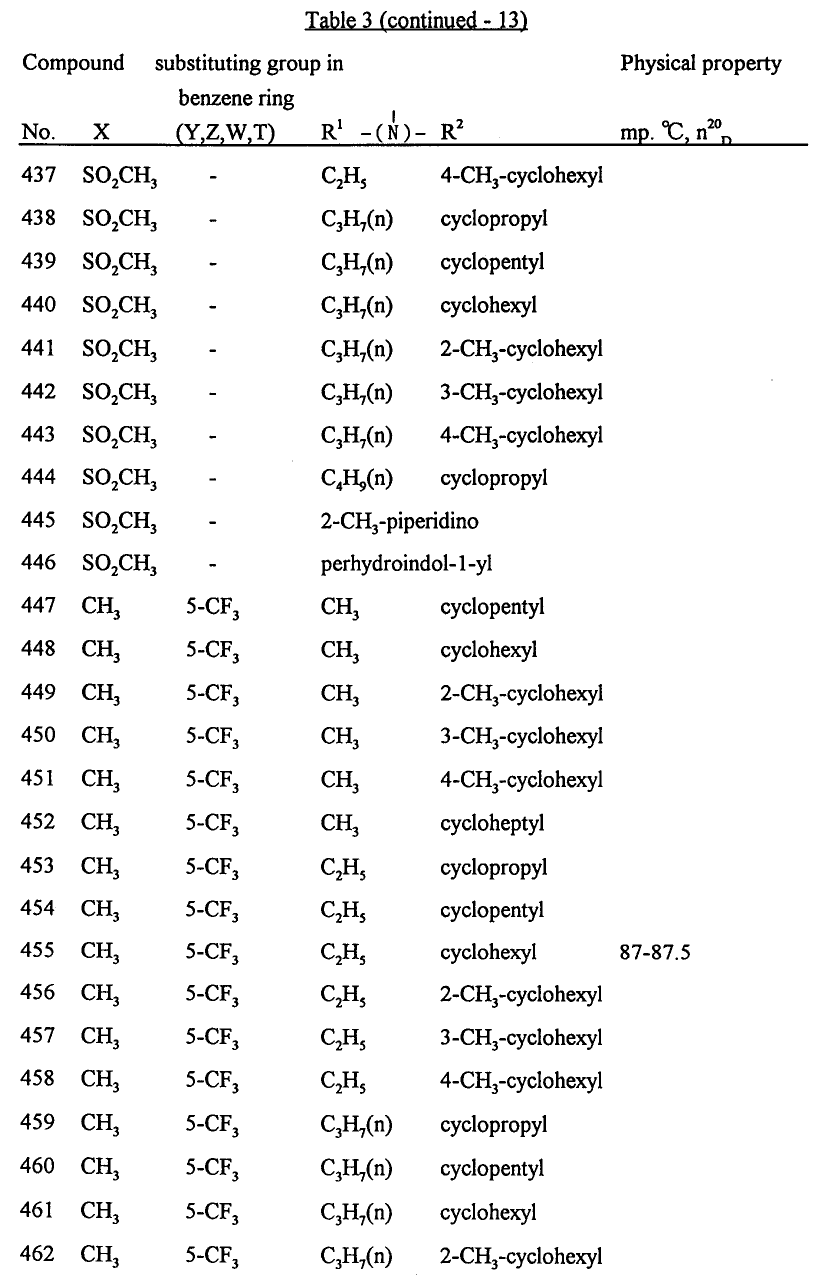

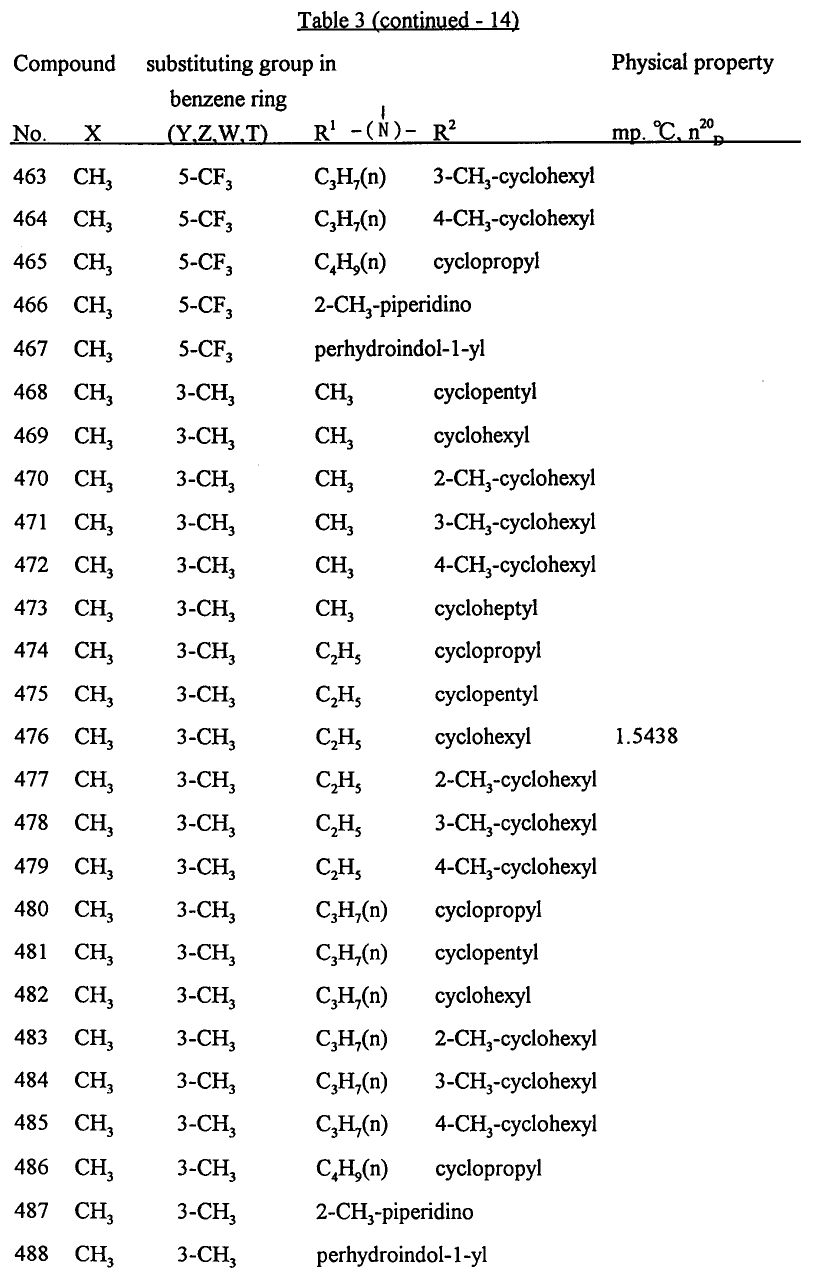

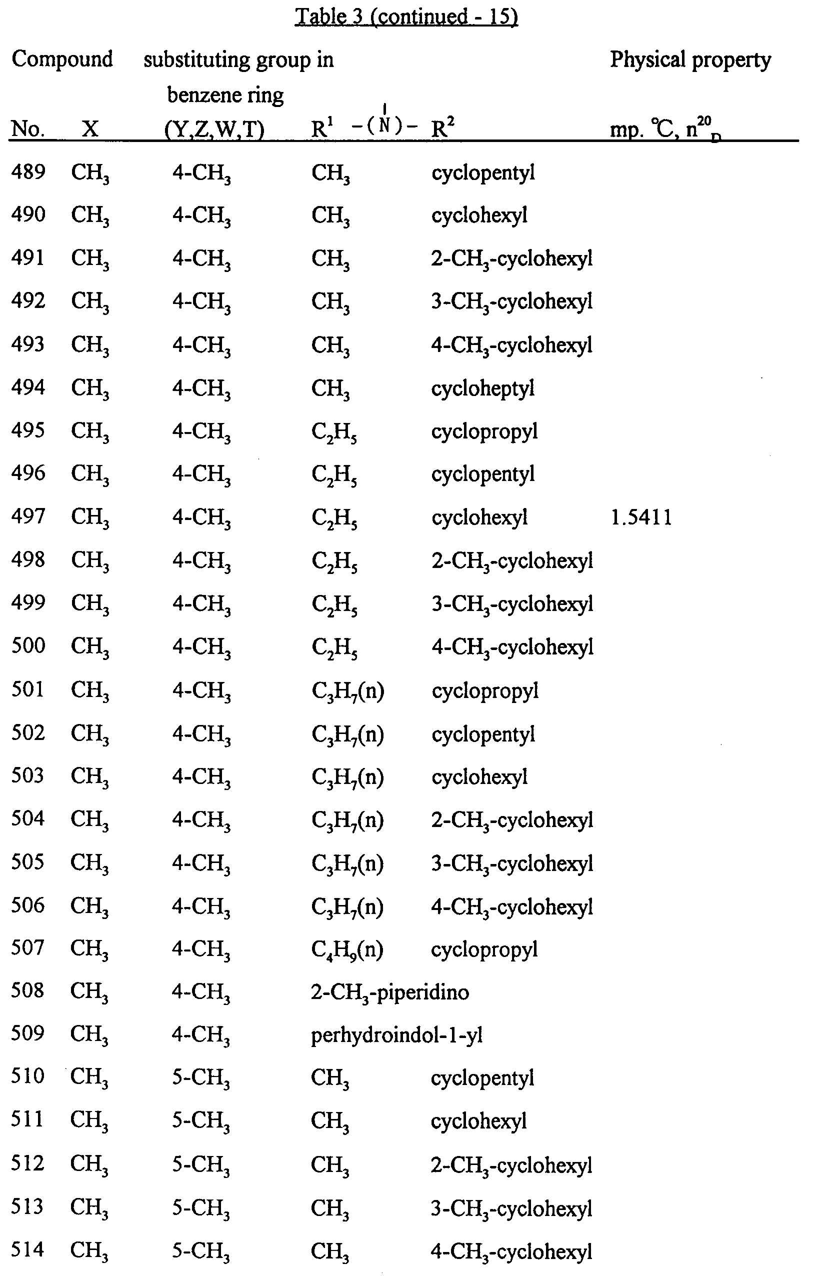

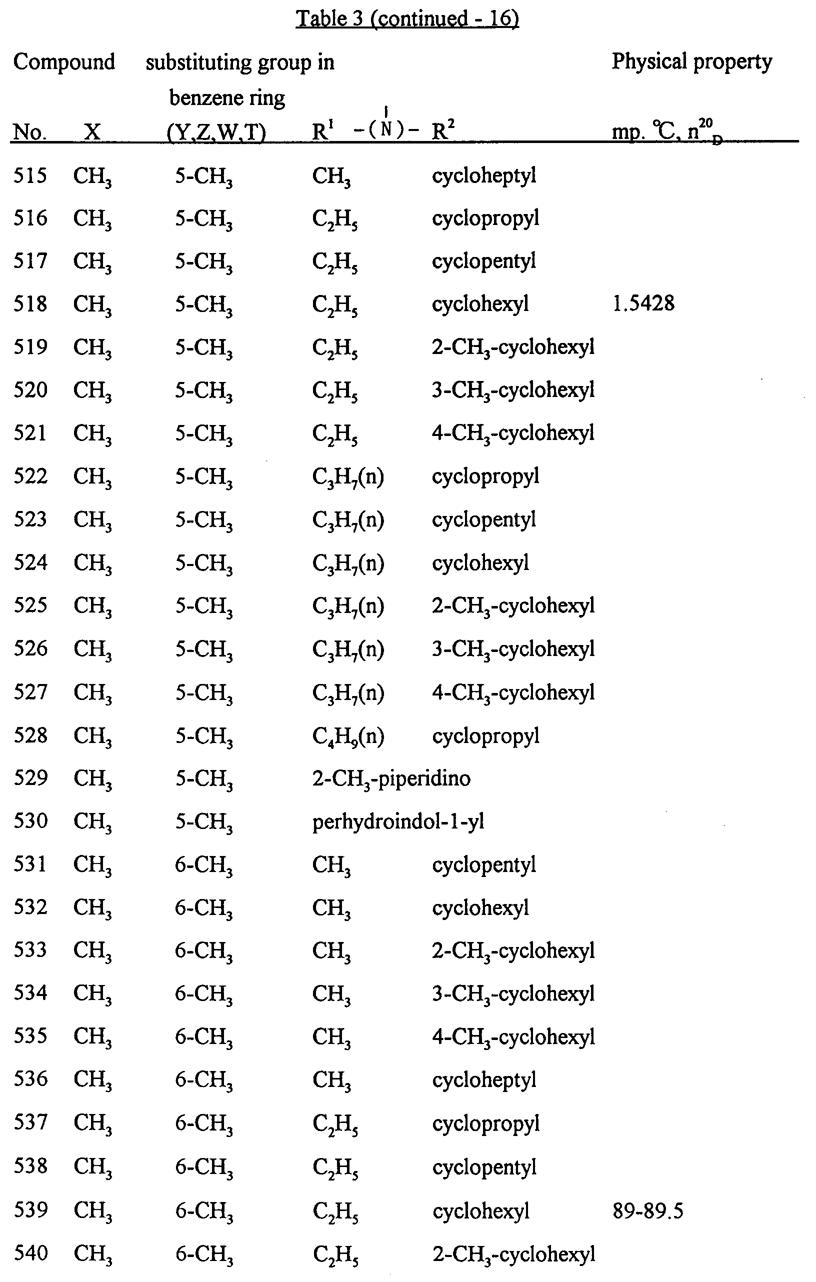

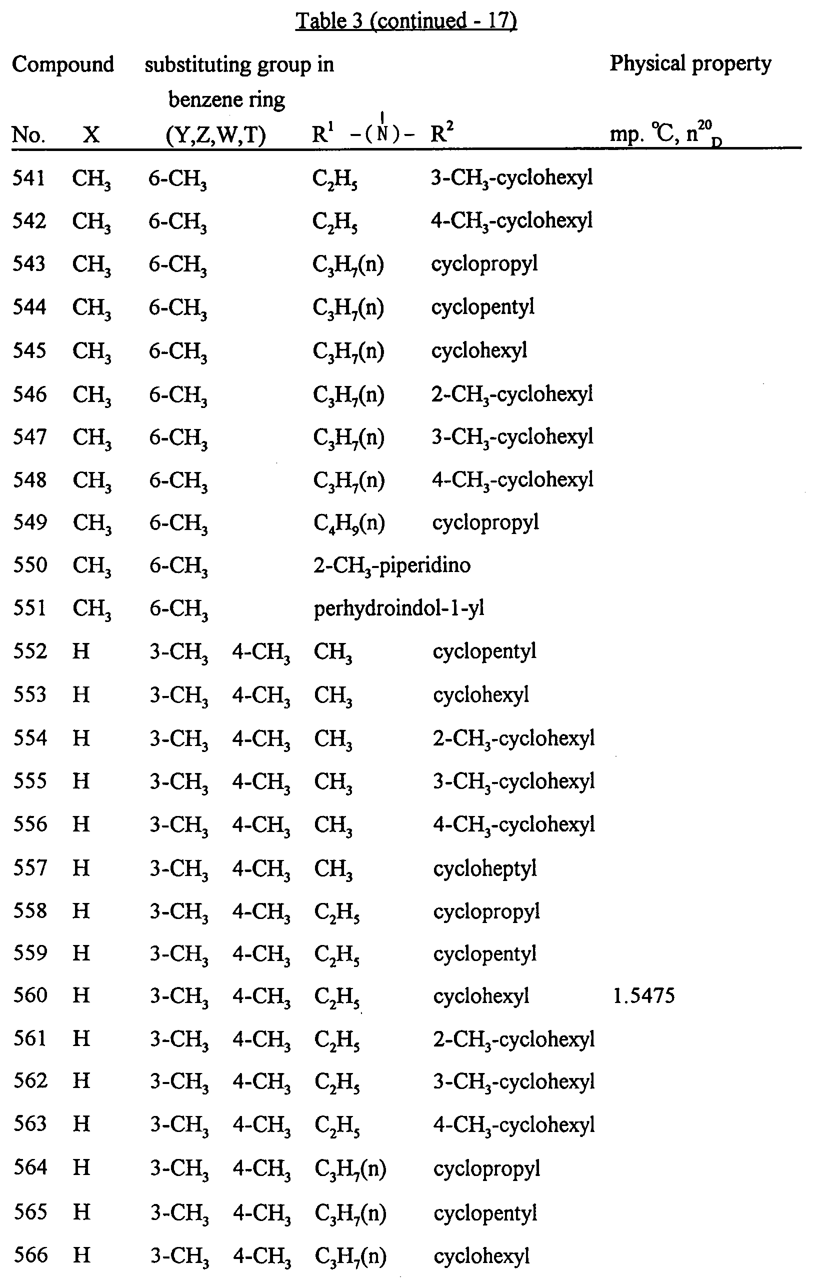

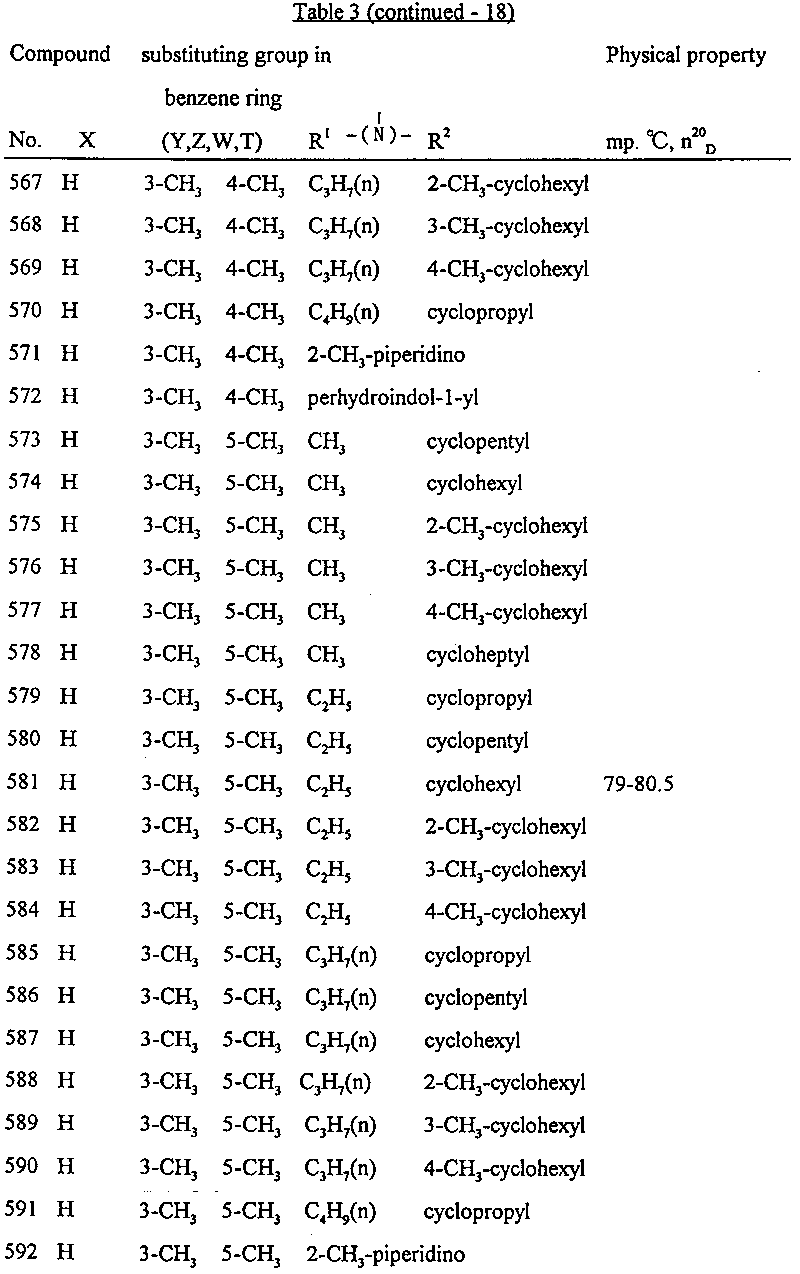

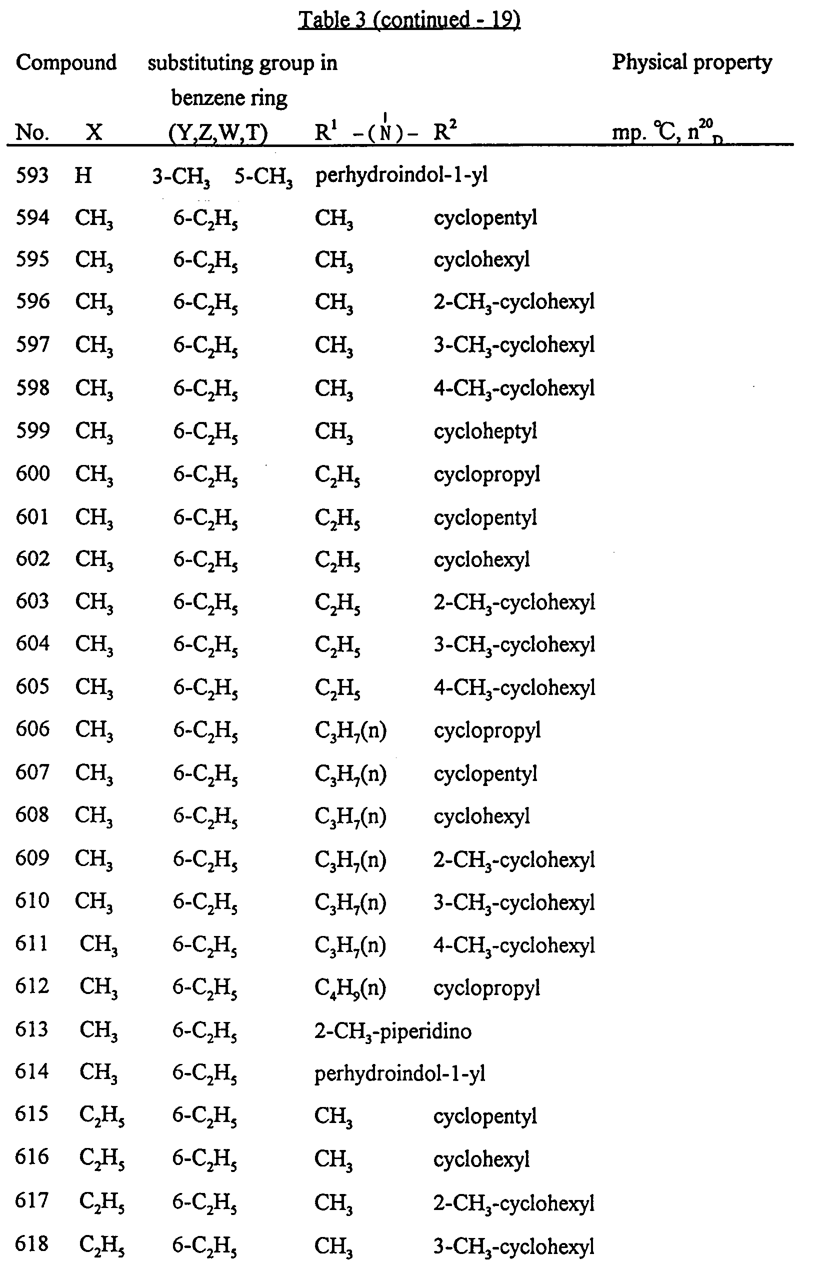

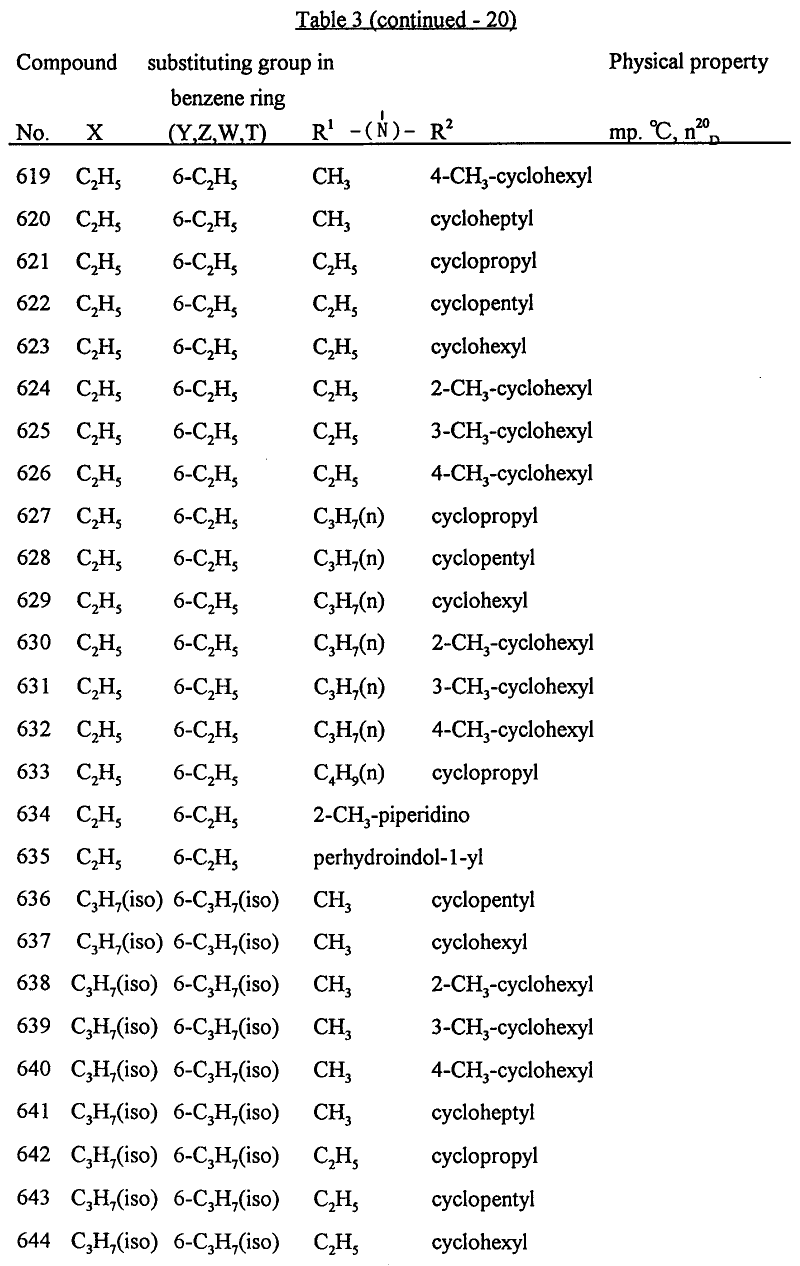

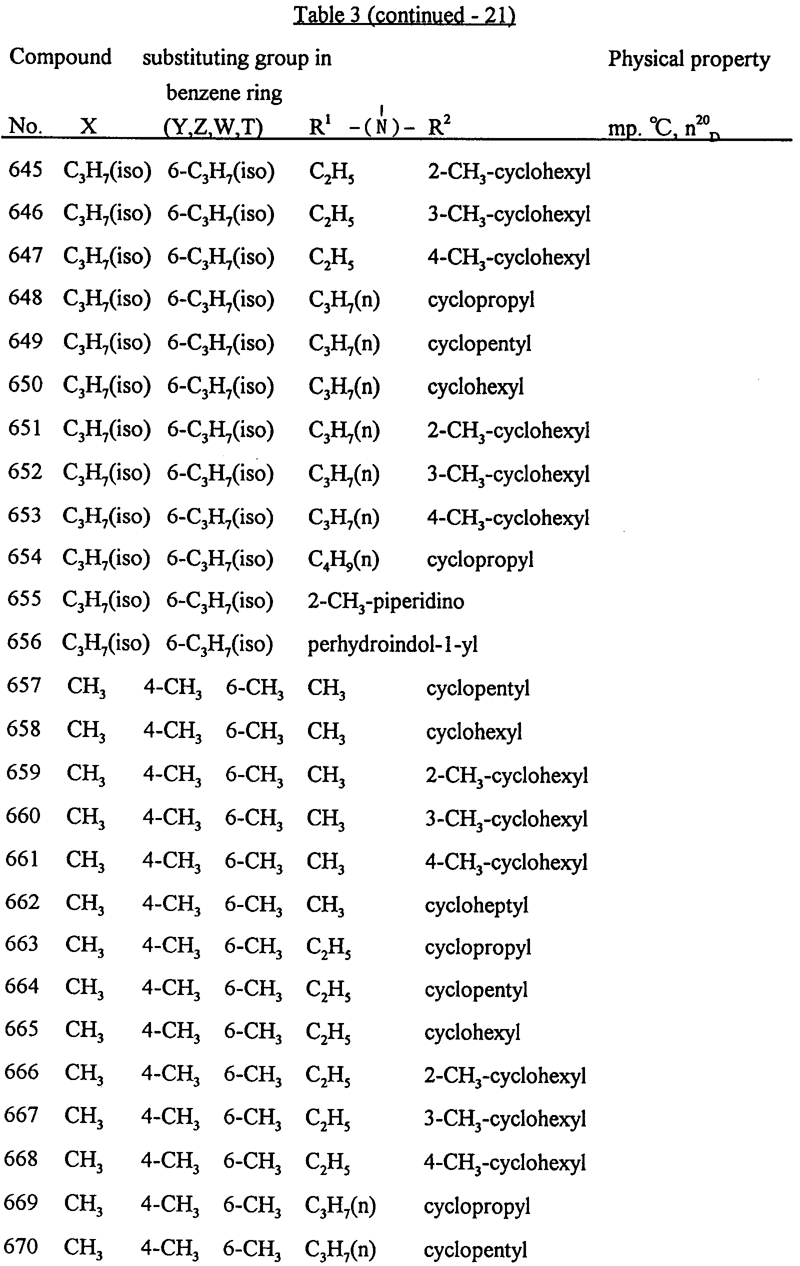

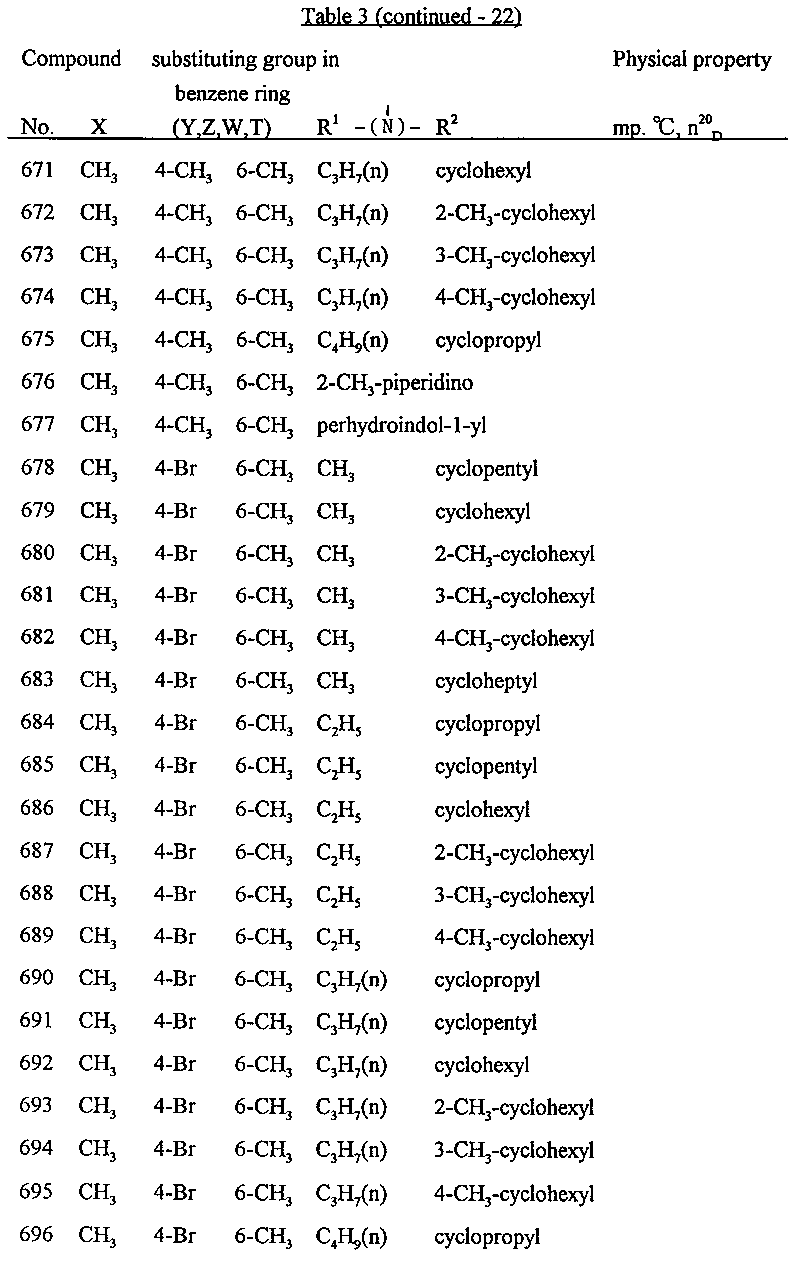

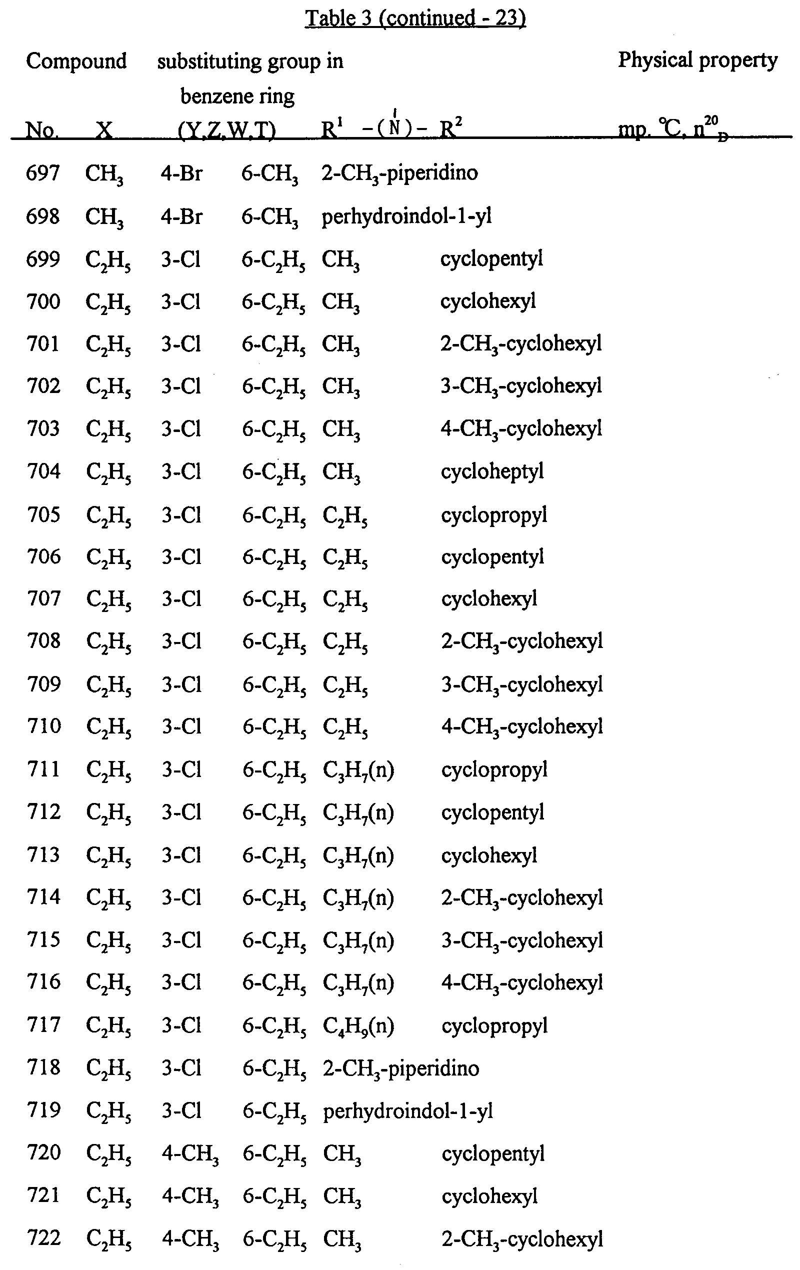

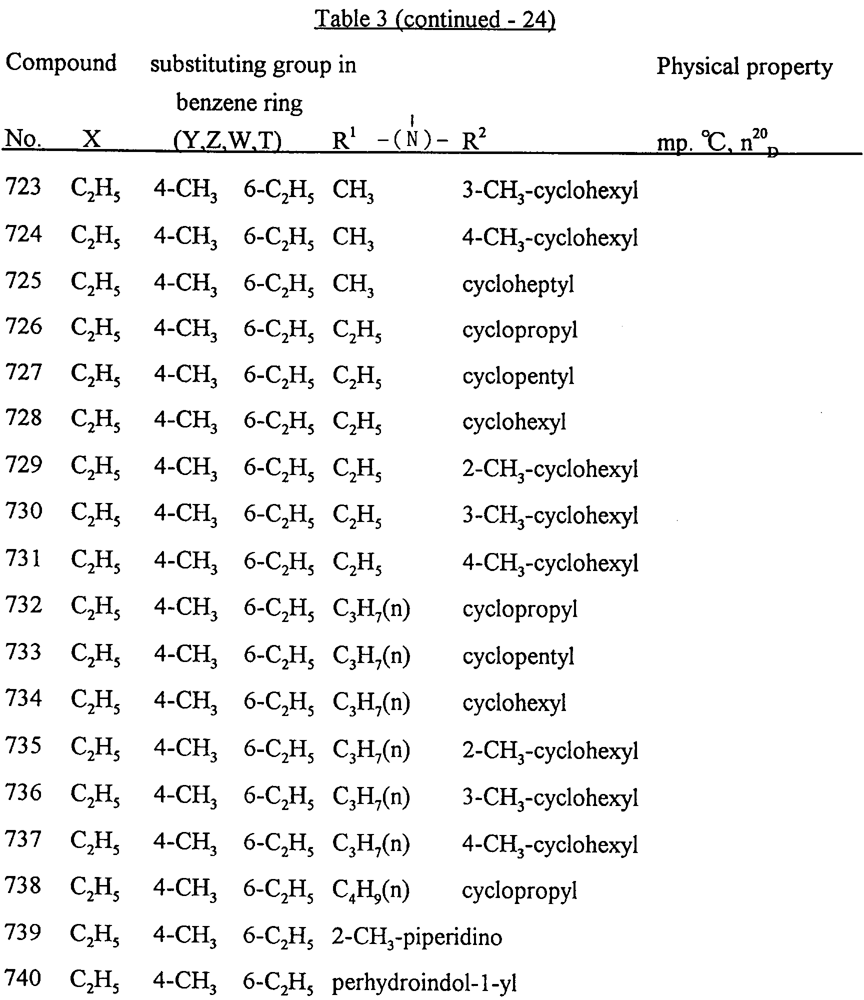

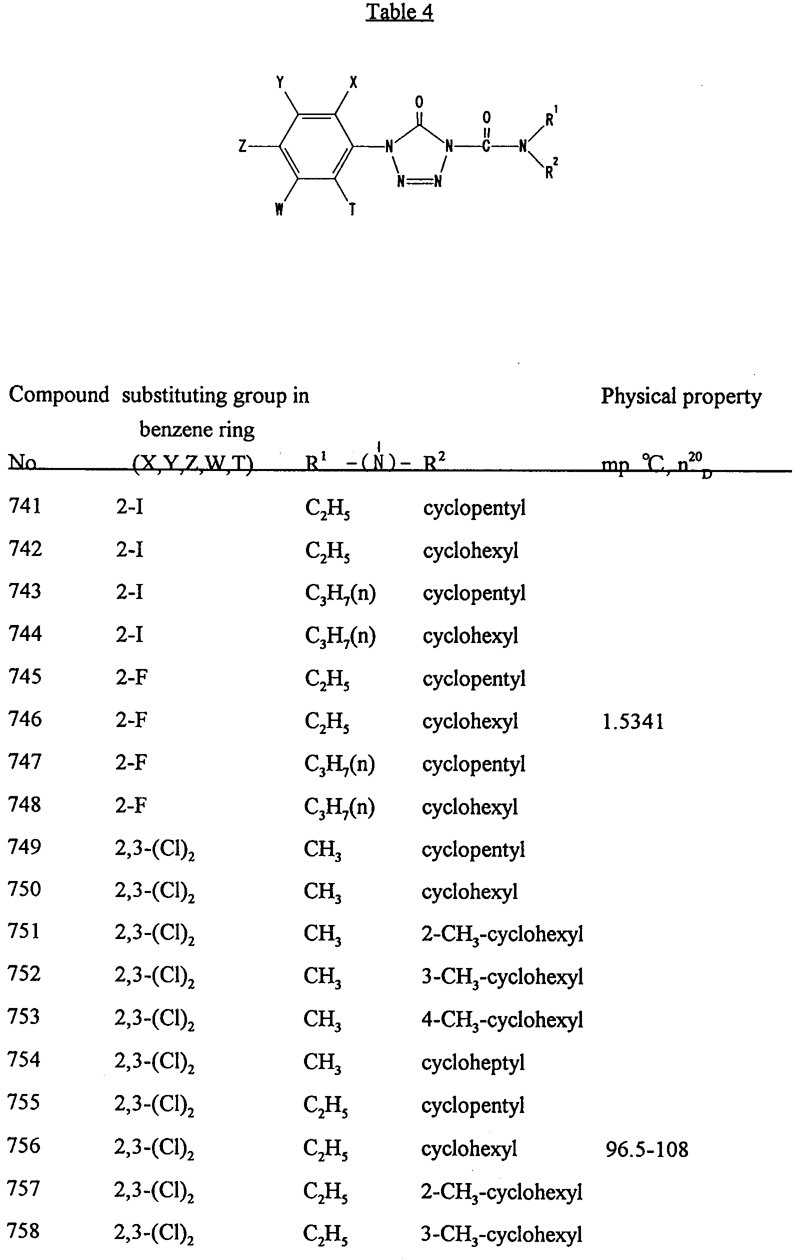

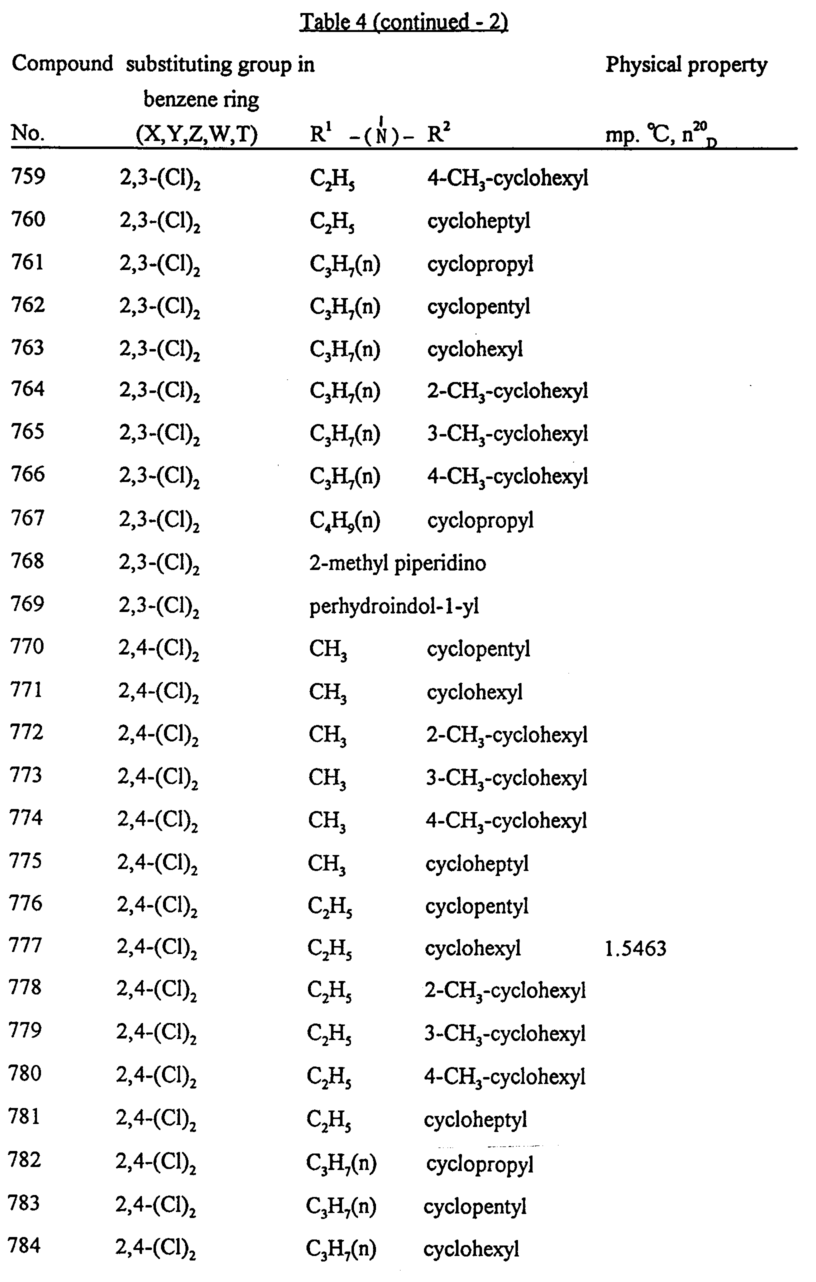

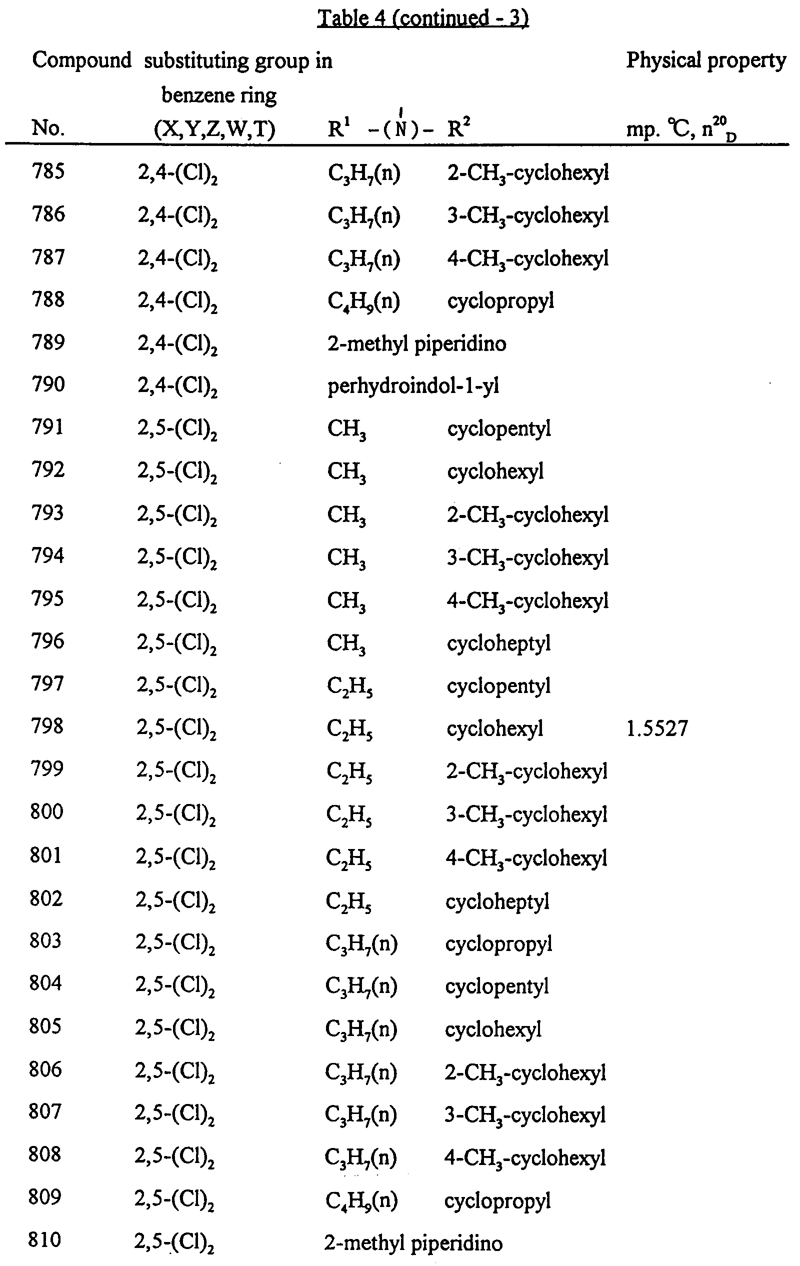

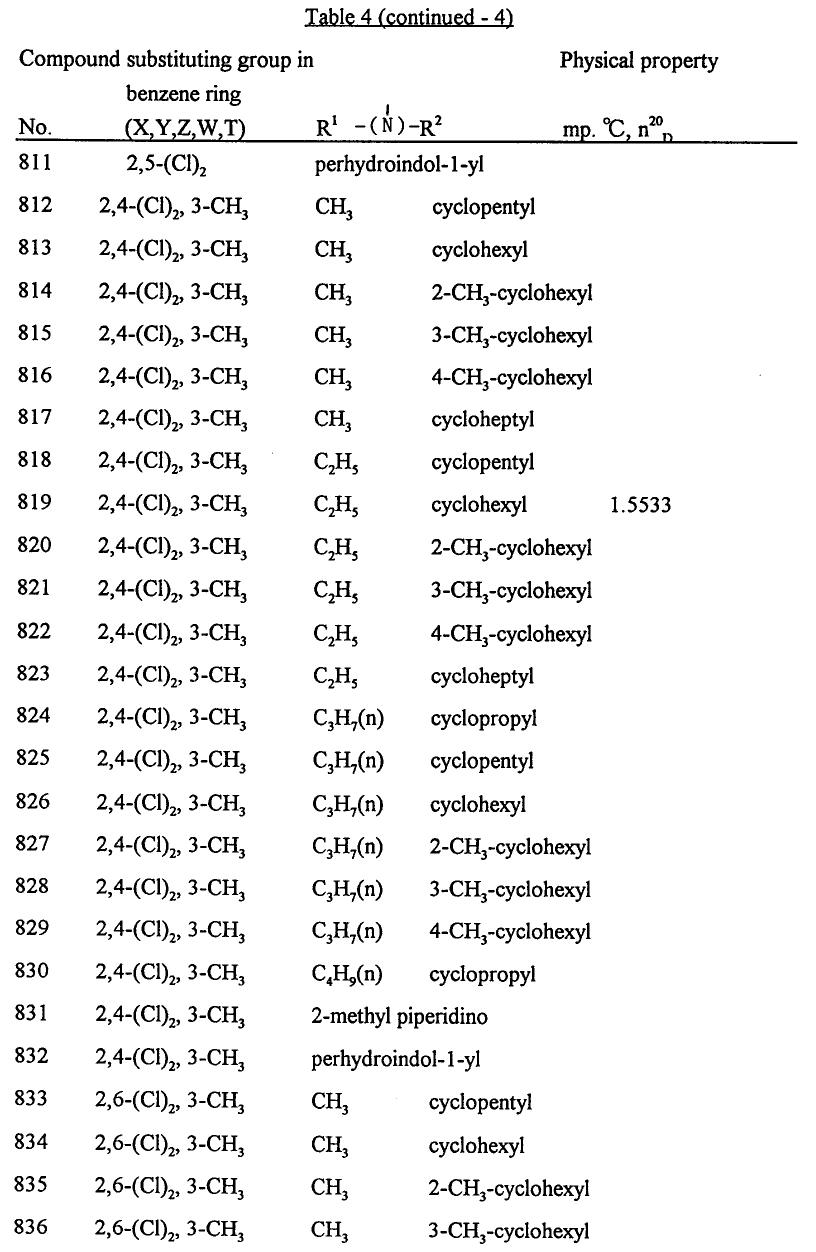

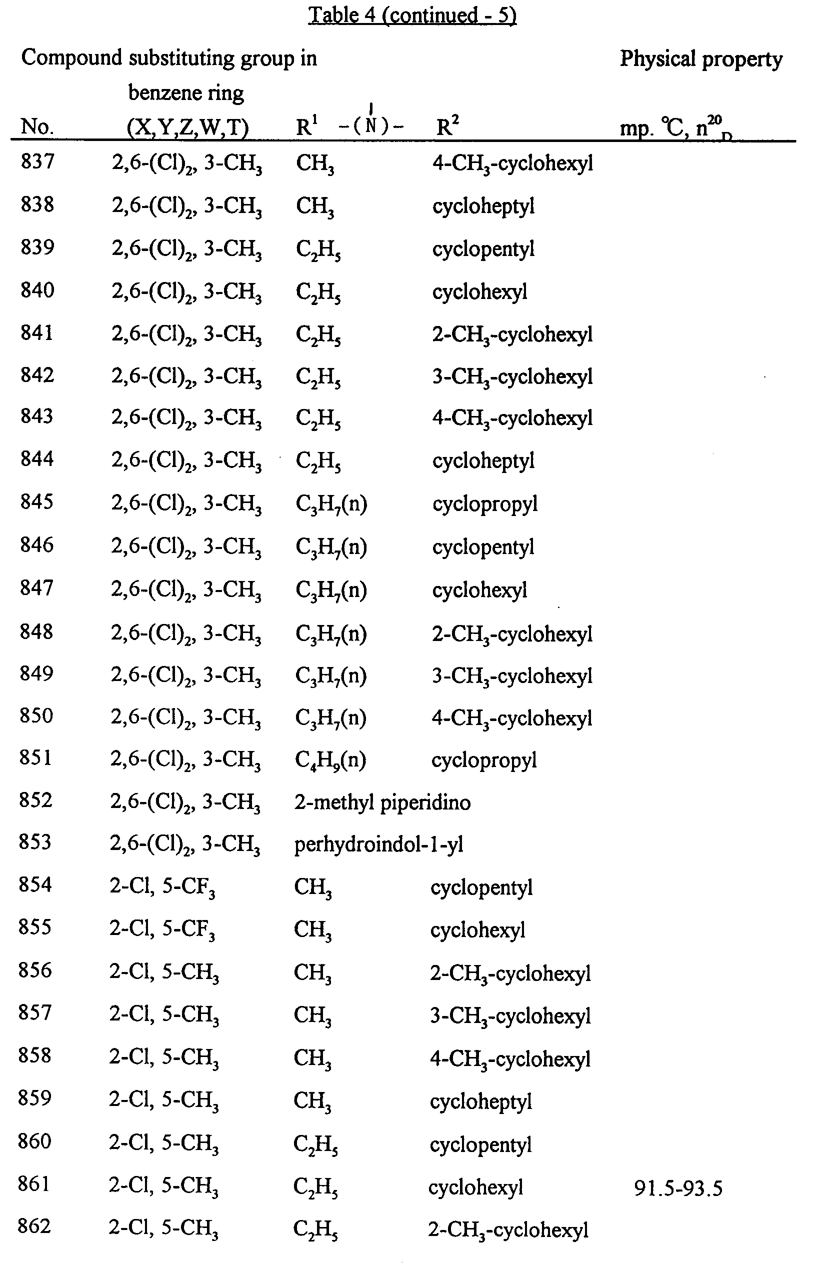

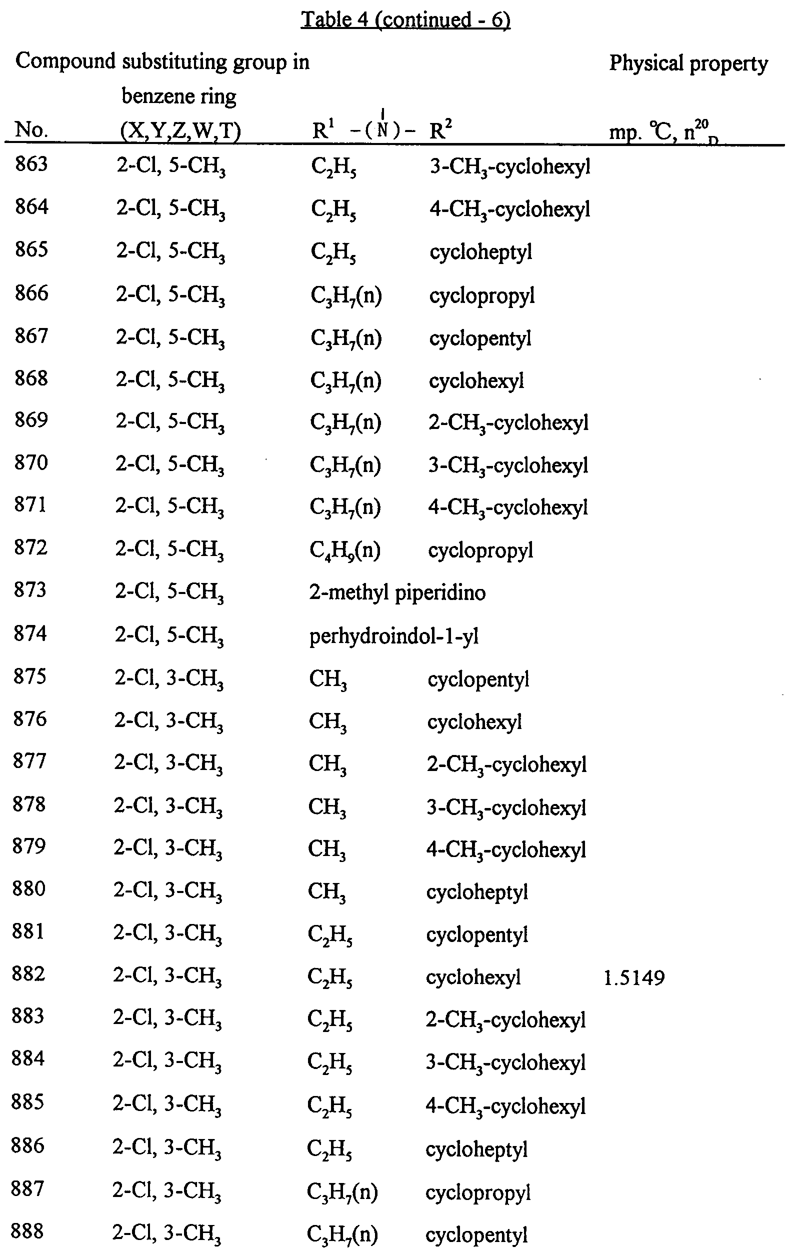

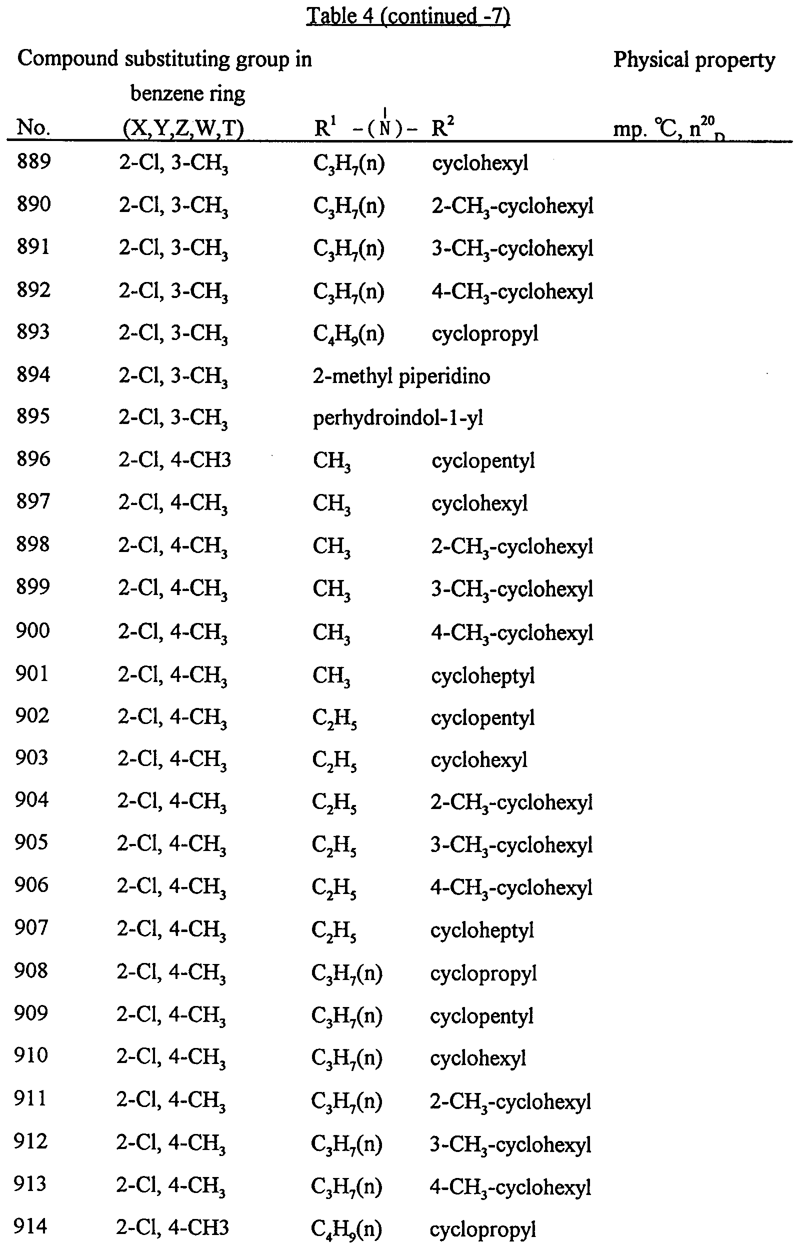

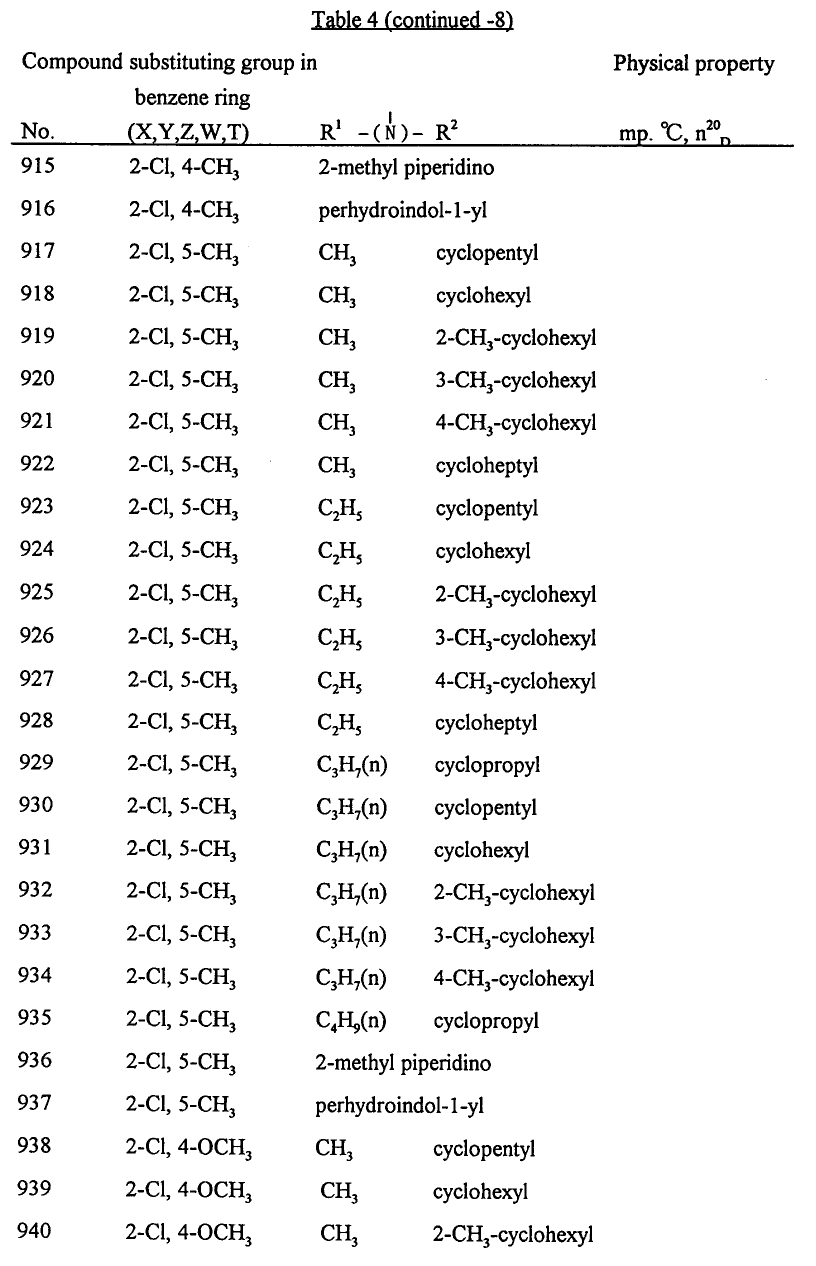

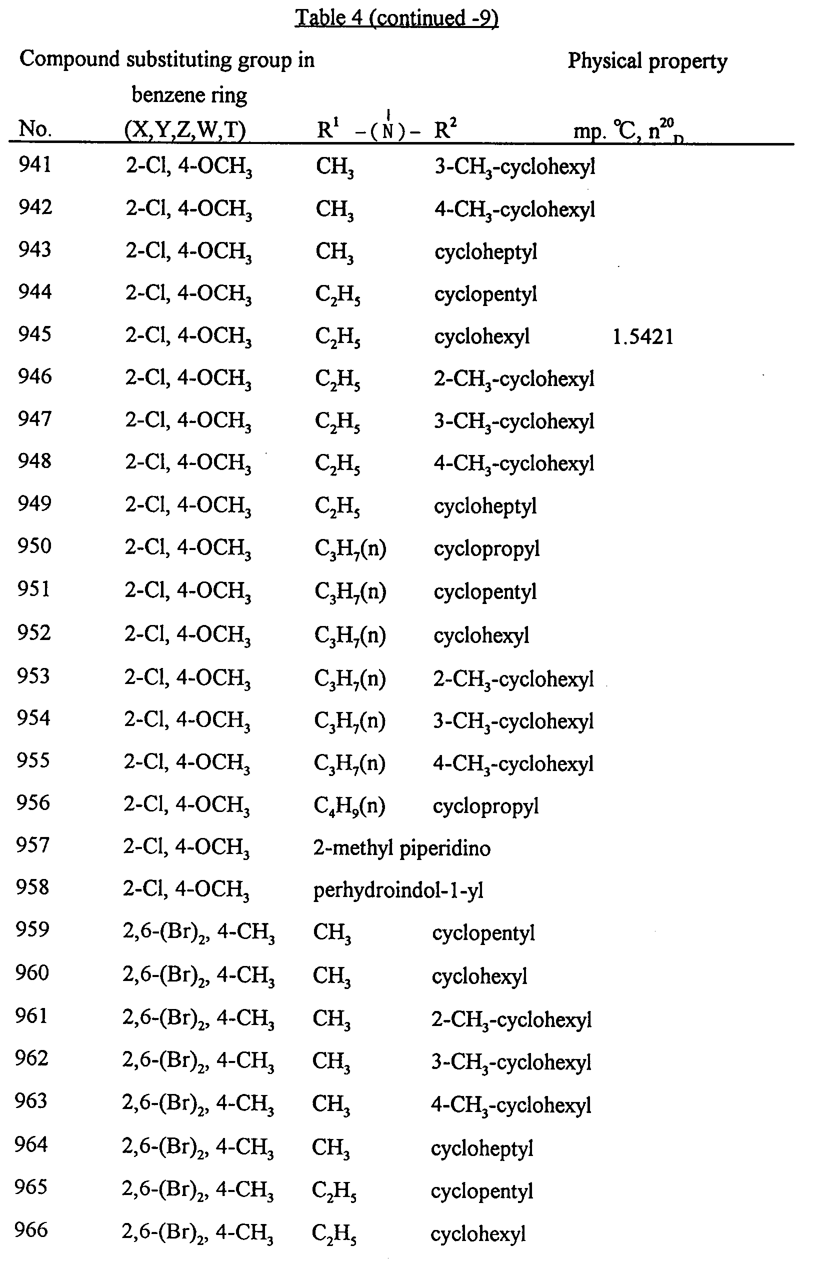

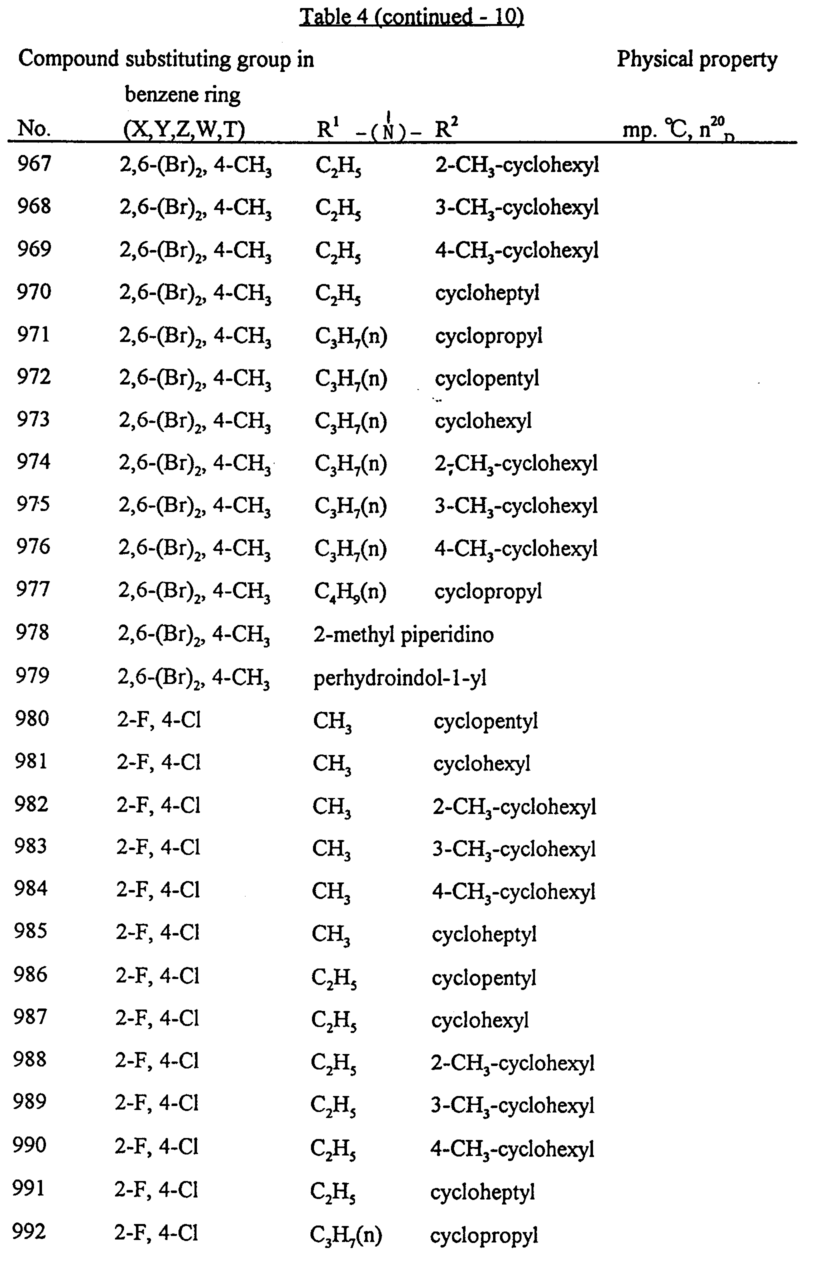

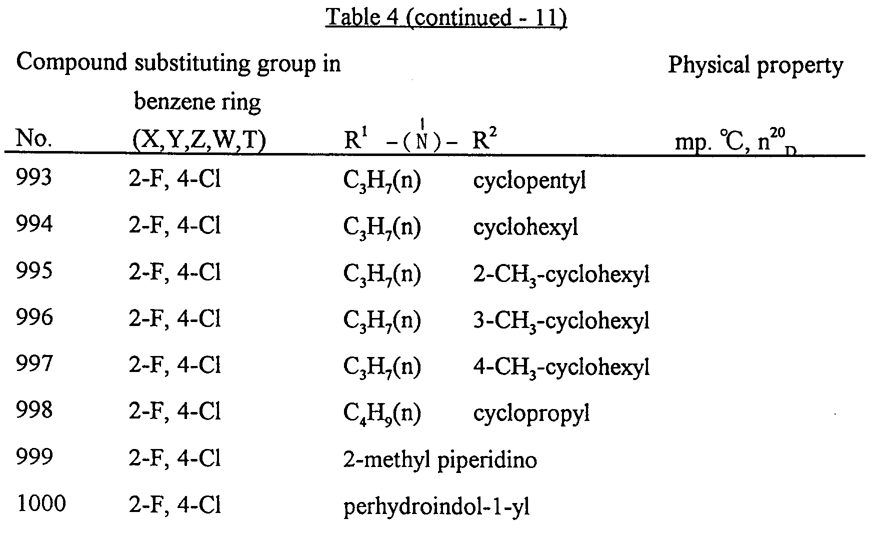

### Test Example 1

### Test of pre-emergence soil treatment against upland field weeds

### Preparing method:

carrier: acetone 5 parts
emulsifier: benzyloxy polyglycol ether 1 part
One part of an active compound and the above amounts of carrier and emulsifier are mixed to obtain an emulsion. A prescribed amount of this emulsion is diluted with water to be subjected to the test below.

### Testing procedure:

In the greenhouse, seeds of Echinochloa, Setaria, Polygonum and Amaranthus lividus were sowed each in the 120 cm² pot filled with soil taken from a cultivated field and covered with soil, and then a prescribed amount of the above testing chemical was uniformly sprayed each on the surface layer of soil in the testing pot. The herbicidal effect was examined on the day after 4 weeks from application. The herbicidal effect was rated as 100% in the case of complete death and as 0% in the case where equivalent growth was observed to the case without treatment.

### Results:

The compound No. 3 exhibited 100% herbicidal effect against Echinochloa and Setaria by application of 1 kg/ha as the effective component and exhibited 80% or more of the herbicidal effect against Polygonum and Amaranthus lividus by application of 2 kg/ha as the effective component.

### Test Example 2

### Test of pre-emergence soil treatment against upland field weeds

### Preparing method:

carrier: acetone 5 parts
emulsifier: benzyloxy polyglycol ether 1 part
One part of an active compound and the above amounts of carrier and emulsifier are mixed to obtain an emulsion. A prescribed amount of this emulsion is diluted with water to be subjected to the test below.

### Testing procedure:

In the greenhouse, seeds of Echinochloa and Amaranthus lividus were sowed each in the 120 cm² pot filled with soil taken from a cultivated field and covered with soil, and then a prescribed amount of the above testing chemical was uniformly sprayed each on the surface layer of soil in the testing pot. The herbicidal effect was examined on the day after 4 weeks from application. The herbicidal effect was rated as 100% in the case of complete death and as 0% in the case where equivalent growth was observed to the case without treatment.

### Results:

The compound Nos. 23, 30, 31, 105, 113, 122, 139, 144, 203, 224, 229, 236, 476, 746, 798, 819, 861, 882 and 945 exhibited 100% herbicidal effect against Echinochloa and Amaranthus lividus by application of 2 kg/ha as the effective component.

### Test Example 3

Test of post-emergence soil treatment against upland field weeds

### Testing procedure:

In the greenhouse, seeds of Echinochloa and Amaranthus lividus were sowed each in the 120 cm² pot filled with soil taken from a cultivated field and covered with soil. After 10 days from sowing and soil-covering (when the weeds were in 2-foliage period on average), each a prescribed amount of the chemical prepared similarly to those in above Test Example 2 was uniformly sprayed on the foliage part of tested plant in the testing pot. After 3 weeks from application, the extent of herbicidal effect was examined.

### Results:

The compound Nos. 23, 30, 31, 105, 113, 122, 139, 144, 151, 161, 203, 245, 266, 371, 455, 476, 497, 539, 746, 777, 819, 861 and 882 exhibited at least 80 % of the herbicidal effect against Echinochloa and Amaranthus lividus by application of 2 kg/ha as the effective component.

### Formulation Example 1 (granules)

Twenty-five parts of water are added to a mixture of 10 parts of Compound No. 3, 30 parts of montmorillonite, 58 parts of talc and 2 parts of lignin sulphonate salt for well kneading followed by granulating in 10-40 mesh using an extrusion type granulator and drying at 40-50°C to obtain granules.

### Formulation Example 2 (granules)

A rotary mixer is charged with 95 parts of a clay mineral particles having 0.2-2 mm of the particle size distribution and 5 parts of Compound No. 30 is sprayed therein with a liquid diluent under rotation for uniformly wetting followed by drying at 40-50°C to give granules.

### Formulation Example 3 (granules)

An emulsion is obtained by mixing 30 parts of Compound No. 25, 55 parts of xylene, 8 parts of polyoxyethylene alkyl phenyl ether and 7 parts of calcium alkylbenzene sulphonate with stirring.

### Formulation Example 4 (wettable powder)

A wettable powder is prepared by crushing and mixing 15 parts of Compound No. 31, 80 parts of a mixture (1 : 5) of "White Carbon" (fine powder of hydrated amorphous silicon oxide) and powdery clay, 2 parts of sodium alkylbenzene sulphonate and 3 parts of a condensate of sodium alkylnaphthalene sulphonate and formaldehyde.

### Formulation Example 5 (wettable granules)

Wettable granules are prepared by thoroughly mixing 20 parts of Compound No. 30, 31 parts of sodium lignin sulphonate, 5 parts of bentonite and 35 parts of calcined diatomaceous earth powder, then adding water and extruding the resultant mixture through a 0.3 mm screen followed by drying.

## Claims

1. Novel tetrazolinones of the formula (I) 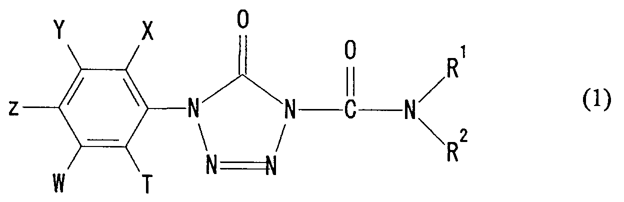 wherein
X is hydrogen, halogen, C₁₋₄ alkyl, C₁₋₂ alkoxy, C₁₋₂ alkylthio, C₁₋₂ alkylsulfonyl, C₁₋₂ alkylsulfinyl or halogeno-C₁₋₂ alkoxy,
Y is hydrogen, halogen, C₁₋₄ alkyl or trifluoromethyl,
Z is hydrogen, halogen, C₁₋₄ alkoxy or halogeno-C₁₋₂ alkoxy,
W is hydrogen, halogen, C₁₋₄ alkyl or trifluoromethyl,
T is hydrogen, halogen or C₁₋₄ alkyl,
R¹ is C₁₋₅ alkyl,
R² is C₃₋₇ cycloalkyl which may optionally be substituted by alkyl, C₅₋₆ cycloalkenyl, a bridged alicyclic group or C₄₋₆ branched alkyl,
and
R¹ and R² may form an optionally substituted cyclic group, together with the nitrogen atom to which they are bonded,
with the proviso that where R² is C₄₋₆ branched alkyl, then the total number of the carbon atoms of R¹ and R² is 7 or 8, and
with the exception of the case where
X is chlorine or bromine,
Y, Z and W are hydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8.

2. The compounds of the formula (I) according to claim 1 wherein
X is hydrogen, fluorine, chlorine, bromine, methyl, methoxy, ethoxy, methylthio, methylsulfonyl, methylsulfinyl, trifluoromethoxy, difluoromethoxy or 2,2,2-trifluoroethoxy,
Y is hydrogen, chlorine, bromine, methyl, ethyl or trifluoromethyl,
Z is hydrogen, fluorine, chlorine, bromine, methoxy, ethoxy or difluoromethoxy,
W is hydrogen, fluorine, chlorine, bromine, methyl, ethyl or trifluoromethyl,
T is hydrogen, fluorine, chlorine, bromine, methyl or ethyl,
R¹ is methyl, ethyl, n-propyl, n-butyl, isobutyl or n-pentyl,
R² is cyclopropyl, C₅₋₇ cycloalkyl which may optionally be substituted by methyl, C₅₋₆ cycloalkenyl, norbornyl, norbornenyl, isobutyl, sec-butyl or 1,2,2-trimethylpropyl, and R¹ and R² may form 2-methylpiperidino, perhydroindol-1-yl or perhydroquinolin-1-yl, together with the nitrogen atom to which they are bonded,
with the proviso that where R² is isobutyl, sec-butyl or 1,2,2-trimethylpropyl, then the total number of the carbon atoms of R¹ and R² is 7 or 8, and
with the exception of the case where
X is chlorine or bromine,
Y, Z and W are bydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8.

3. The compounds of the formula (I) according to claim 1
wherein
X is chlorine, bromine, methyl, methoxy, ethoxy, methylthio, methylsulfonyl or difluoromethoxy,
Y is hydrogen, chlorine, bromine or methyl,
Z is hydrogen, chlorine, bromine or methoxy,
W is hydrogen, chlorine, bromine, methyl or trifluoromethyl,
T is hydrogen, chlorine, bromine or methyl,
R¹ is methyl, ethyl, n-propyl, n-butyl, isobutyl or n-pentyl,
R² is cyclopropyl, C₅₋₆ cycloalkyl which may optionally be substituted by methyl, 1-cyclopentenyl, 2-cyclopentenyl, 1-cyclohexenyl, 2-cyclohexenyl, 2-norbornyl, 5-norbornen-2-yl, isobutyl, sec-butyl or 1,2,2-trimethylpropyl, and
R¹ and R² may form perhydroindol-1-yl or perhydroquinolin-1-yl, together with the nitrogen atom to which they are bonded,
with the proviso that where R² is isobutyl, sec-butyl or 1,2,2-trimethylpropyl, then the total number of the carbon atoms of R¹ and R² is 7 or 8, and
with the exception of the case where
X is chlorine or bromine,
Y, Z and W are hydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8.

4. A process for the preparation of the compounds of the formula (I) 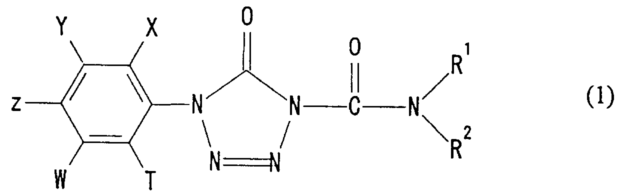 wherein
X is hydrogen, halogen, C₁₋₄ alkyl, C₁₋₂ alkoxy, C₁₋₂ alkylthio, C₁₋₂ alkylsulfonyl, C₁₋₂ alkylsulfinyl or halogeno-C₁₋₂ alkoxy,
Y is hydrogen, halogen, C₁₋₄ alkyl or trifluoromethyl,
Z is hydrogen, halogen, C₁₋₄ alkoxy or halogeno-C₁₋₂ alkoxy,
W is hydrogen, halogen, C₁₋₄ alkyl or trifluoromethyl,
T is hydrogen, halogen or C₁₋₄ alkyl,
R¹ is C₁₋₅ alkyl,
R² is C₃₋₇ cycloalkyl which may optionally be substituted by alkyl, C₅₋₆ cycloalkenyl, a bridged alicyclic group or C₄₋₆ branched alkyl,
and
R¹ and R² may form an optionally substituted cyclic group, together with the nitrogen atom to which they are bonded,
with the proviso that where R² is C₄₋₆ branched alkyl, then the total number of the carbon atoms of R¹ and R² is 7 or 8, and
with the exception of the case where
X is chlorine or bromine,
Y, Z and W are hydrogen,
T is hydrogen, chlorine, bromine or methyl,
R¹ is ethyl or n-propyl, and
R² is C₅₋₆ cycloalkyl, provided that the total number of the carbon atoms of R¹ and R² is 7 or 8,
characterized in that
compounds of the formula (II) 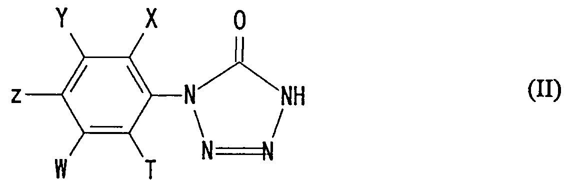 wherein
X, Y, Z, W and T have the same meanings as stated above, are reacted with compounds of the formula (III) 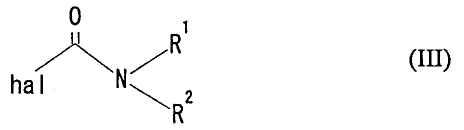 wherein
R¹ and R² have the same meanings as stated above, and hal is a leaving group such as chlorine and bromine,
in the presence of an inert solvent, and if appropriate, in the presence of an acid binder.

5. Herbicidal compositions characterized in that they contain at least one tetrazolinone of the formula (I) according to claim 1.

6. Process for combating weeds, characterized in that tetrazolinones of the formula (I) according to claim 1 are allowed to act on the weeds and/or their habitat.

7. Use of tetrazolinones of the formula (I) according to claim 1 for combating weeds.

8. Process for the preparation of herbicidal compositions, characterized in that tetrazolinones of the formula (I) according to claim 1 are mixed with extenders and/or surface active agents.
